(19)

**Europäisches Patentamt**
**European Patent Office**
**Office européen des brevets**

(11) **EP 4 752 220 A1**

(12) **EUROPEAN PATENT APPLICATION**

(43) Date of publication:
**03.06.2026 Bulletin 2026/23**

(21) Application number: **24383305.0**

(22) Date of filing: **02.12.2024**

(51) International Patent Classification (IPC):
*C12N 9/42* $^{(2006.01)}$    *C07K 14/375* $^{(2006.01)}$
*C12P 19/04* $^{(2006.01)}$    *C12P 19/14* $^{(2006.01)}$
*C08B 15/08* $^{(2006.01)}$    *C09D 11/08* $^{(2006.01)}$
*C09D 11/102* $^{(2014.01)}$    *C09D 11/14* $^{(2006.01)}$
*C09D 11/52* $^{(2014.01)}$

(52) Cooperative Patent Classification (CPC):
**C12N 9/2437; C07K 14/375; C08B 15/02;
C08B 15/08; C09D 11/14; C09D 11/52;
C12P 19/04; C12P 19/14;** C12Y 302/01004

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**GE KH MA MD TN**

(71) Applicants:
• **Asociación Centro de Investigación Cooperativa
en Biociencias - CIC bioGUNE
48160 Derio (ES)**
• **Aalto University Foundation SR
00076 Aalto (FI)**

(72) Inventors:
• **PÉREZ JIMÉNEZ, Raúl
E-48160 Derio, Vizcaya (ES)**
• **JABALERA RUZ, Ylenia
E-48160 Derio, Vizcaya (ES)**
• **MASTER, Emma
FI-00076 AALTO (FI)**
• **DAHIYA, Deepika
FI-00076 AALTO (FI)**

(74) Representative: **ABG Intellectual Property Law,
S.L.
Avenida de Burgos, 16D
Edificio Euromor
28036 Madrid (ES)**

Remarks:
The complete document including Reference
Table(s) and the Sequence Listing(s) can be
downloaded from the EPO website

(54) **COMBINATION FOR CRYSTALLINE NANOCELLULOSE PRODUCTION**

(57)    The invention relates to a combination comprising an enzyme comprising an endoglucanase catalytic domain and a loosening or a loosenin-like protein, as well as to a method for producing crystalline nanocellulose.

The invention also relates to crystalline nanocellulose obtainable by said method and to a method for preparing a conductive ink using said crystalline nanocellulose.

**EP 4 752 220 A1**

**Description**

## TECHNICAL FIELD OF THE INVENTION

The invention relates to the field of crystalline nanocellulose, its applications and the methods for producing crystalline nanocellulose.

## BACKGROUND OF THE INVENTION

Nanocellulose is a high-value material obtained from lignocellulosic biomass. Nanocellulose forms are derived from the hierarchical organization of cellulose, resulting in different types of materials, including cellulose nanocrystals (CNC). CNCs are small crystalline particles, typically ranging from 100 to 500 nm in length. While acidic hydrolysis has been extensively used for CNC production, the modifications it causes to the physicochemical properties of CNC (e.g., through the addition of sulfate groups) have driven interest in enzymatic production. In most cases so far, the enzymatic production of CNCs has employed complex enzyme cocktails; however, simplifying the enzyme system is expected to ensure the reproducible production of CNC preparations. For example, an ancestral endoglucanase from the last Firmicutes common ancestor (LFCA_EG), which diverged over 3 billion years ago, has been proposed as a solution to this challenge (Barruetabeña, N. et al., *Communications Chemistry* 2019, 2 (1), 76). Ancestral bacterial endoglucanases have demonstrated high activity and promiscuity over different substrates, and in contrast to their present-day descendants from *Bacillus subtilis* or other endoglucanase forms, have shown the ability to produce pure nanocellulose nanocrystals in high yield (EnCNC) when combined with a carbohydrate-binding module (CBM).

The catalytic domain of an ancestral endoglucanase (LFCA_EG) combined with a present-day family 30 carbohydrate-binding module (CBM30) from *C. thermocellum* (LFCA_EG + CBM) was previously shown to produce cellulose nanocrystals at higher yield and better aspect ratio than those produced by the LFCA_EG without CBM (Alonso-Lerma, B. et al., Communications Materials 2020, 1 (1), 57). However, despite the improved performance of LFCA_EG over the parent EG, the resulting CNC was still approximately two times longer than CNCs prepared using acid hydrolysis.

EnCNC has been used for various biotechnological applications, such as forming stable matrices for cell growth and for the preparation of conductive printable inks made by graphite exfoliation. Notably, conductive inks currently contain toxic metals such as copper or high-cost metals like silver or gold. By comparison, conductive carbon-based materials (graphite and cellulose) stand out as a low cost and eco-friendly alternative. Whereas EnCNC retains the physicochemical properties of cellulose, EnCNC crystal length tend to exceed 400 nm while acid hydrolysis CNC (AcCNC) tend to be ~ 200 nm and are therefore more effective in graphite exfoliation.

Therefore, there is a need of improved methods for producing CNC with enhanced properties that can be used for multiple biotechnological applications, including the preparation of graphene-based conductive inks.

## SUMMARY OF THE INVENTION

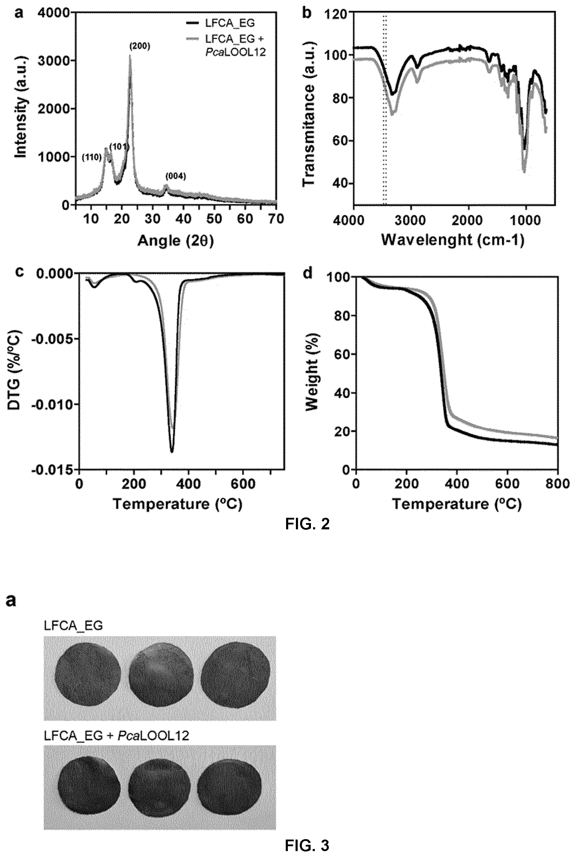

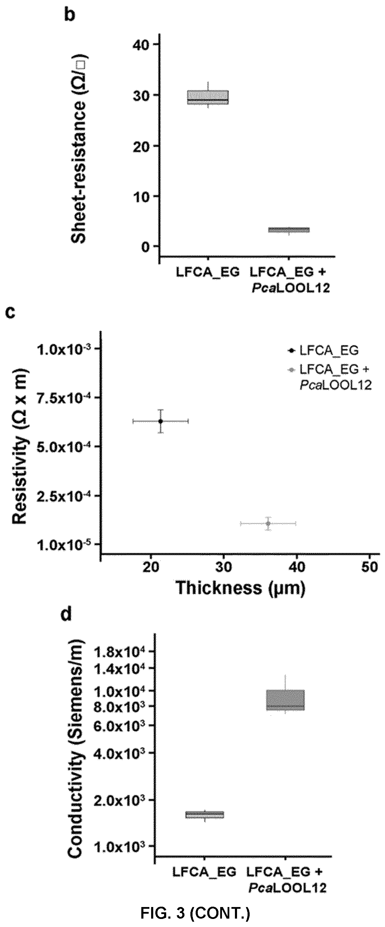

The present invention is based on the unexpected discovery that the so-called ancestral endoglucanases when combined with loosenin or loosenin-like proteins are able produce crystalline nanocellulose (CNC) with a better yield than the ancestral endoglucanases alone (Table 2). Additionally, the resulting CNC has improved properties over the CNC produced by using the ancestral endoglucanases in the absence of loosenin, in particular, smaller size and better morphology (Fig. 2) and improved thermostability (Table 4). Furthermore, when the CNC obtained by the combination of the ancestral endoglucanases with the loosenin-like protein PcaLOOL12 was used for preparing a conductive graphene-based ink, the resulting product had lower resistivity and increased conductivity compared to a an ink obtained with the ancestral endoglucanase alone (Fig. 3).

Therefore, in a first aspect, the invention relates to a combination comprising:

(i) an enzyme comprising an endoglucanase catalytic domain, wherein the endoglucanase catalytic domain comprises a sequence selected from the group consisting of SEQ ID NO: 1, SEQ ID NO: 2, SEQ ID NO: 3, SEQ ID NO: 4 and a functionally equivalent variant thereof that substantially maintains its endoglucanase activity and

(ii) a loosenin or a loosenin-like protein.

In a second aspect, the invention relates to a method for producing crystalline nanocellulose comprising contacting a substrate comprising cellulose with the combination according to any one of claims 1 to 7 under suitable conditions for hydrolyzing cellulose.

In a third aspect, the invention relates to crystalline nanocellulose obtained by the method of the second aspect.

In a fourth aspect, the invention relates to a method for preparing a graphene-based conductive ink comprising the following steps:

(i) mixing the crystalline nanocellulose according to claim 10 with graphite and a first polar solvent, wherein the weight ratio of the crystalline nanocellulose to graphite is comprised from 1:100 to 1:20;

(ii) homogenising the mixture provided in step (i);

(iii) adding to the mixture obtained in step (ii) a second polar solvent having a boiling point boiling point equal to or higher than the first polar solvent provided in step (i); and

(iv) removing substantially all the first polar solvent provided in step (i) while leaving substantially all the second polar solvent added in step (iii).

In fifth aspect, the invention relates to a conductive ink obtainable by the method of the fourth aspect.

## BRIEF DESCRIPTION OF THE FIGURES

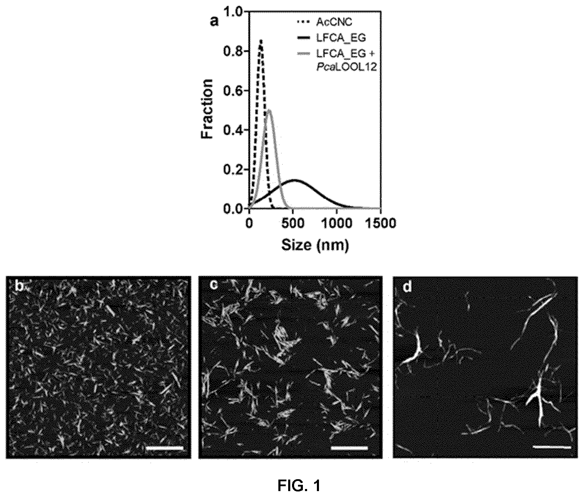

**Figure 1. Size characterization of CNC.** (a) Comparison of size distribution of nanocellulose produced with enzymatic hydrolysis (LFCA_EG and LFCA_EG + PcaLOOL12) and AcCNC. The size (length) distribution was calculated from at least 100 particles from each condition. AFM images of (b) AcCNC, (c) LFCA_EG + PcaLOOL12 and (d) LFCA_EG nanoparticles. Scale bar = 1 $\mu$m.

**Figure 2. Nanocellulose physicochemical characterization.** (a) X-ray diffraction analysis of cellulose crystals produced by LFCA_EG and LFCA_EG + PcaLOOL12 after 24 h. Diffractograms characteristic of cellulose I are shown in (b). FTIR spectra from LFCA_EG and LFCA_EG + PcaLOOL12 CNCs. Peaks at 3486 and 3441 cm$^{-1}$ (characteristics of cellulose type II crystals) are marked with dotted lines. (c) Thermogravimetric analysis curves of filter paper, EnCNCs produced by LFCA_EG and LFCA_EG + PcaLOOL12 after 24 h hydrolysis. (d) Weight loss curves and derivative from TGA curves.

**Figure 3. Electrical properties of printed tags using conductive inks.** (a) Photograph of printed circles with conductive inks. (a) Boxplot of conductivity and (b) sheet-resistance of printed tags. (d) 2D representation of resistivity versus thickness for printed tags using LFCA_EG and LFCA_EG + *Pca*LOOL12 inks. Error bars indicate the standard deviation for both resistivity and thickness measurements.

## DETAILED DESCRIPTION OF THE INVENTION

### *Combination of the invention*

In a first aspect, the invention relates to a combination comprising:

(iii) an enzyme comprising an endoglucanase catalytic domain, wherein the endoglucanase catalytic domain comprises a sequence selected from the group consisting of SEQ ID NO: 1, SEQ ID NO: 2, SEQ ID NO: 3, SEQ ID NO: 4 and a functionally equivalent variant thereof that substantially maintains its endoglucanase activity and

(iv) a loosenin or a loosenin-like protein.

The term "combination", as used herein, refers to any composition-of-matter which contains, either jointly or separately, components (i) and (ii). The term combination includes a combined mixture composed of separate formulations of the components (i) and (ii), such as a "tank-mix", and a composition in which all the components form part of the same mixture. Whenever the combination is provided as a mixture of all components, it will be understood that, when in use, all the components will be used simultaneously. However, if those embodiments wherein the different components of the combination are formulated in different containers, then said components may be used simultaneously, sequentially or separately. Whenever the components of the combination are provided as a mixture of components (i) and (ii), then the terms "combination" and "composition" can be used interchangeably. In a particular embodiment, the combination is a composition comprising components (i) and (ii).

Component (i) of the combination of the invention is an enzyme comprising an endoglucanase catalytic domain, wherein the endoglucanase catalytic domain comprises a sequence selected from the group consisting of SEQ ID NO: 1, SEQ ID NO: 2, SEQ ID NO: 3, SEQ ID NO: 4 and a functionally equivalent variant thereof that substantially maintains its endoglucanase activity.

The term "enzyme comprising an endoglucanase catalytic domain", or "endoglucanase" as used herein, refers to an enzyme that randomly cleaves (1-4)-$\beta$-D-glucosidic links in cellulose, lichenin and cereal $\beta$-D-glucans, thereby creating new chain ends. Endoglucanases also hydrolyse 1,4-linkages in $\beta$-D-glucans also containing 1,3-linkages. It has been classified by the Nomenclature Committee of the International Union of Biochemistry and Molecular Biology as EC 3.2.1.4. Endoglucanases derived from bacteria have a catalytic domain and a carbohydrate binding module attached by a linker or linking domain. However, in the present invention, the term "endoglucanase" is not restricted to enzymes comprising a carbohydrate binding motive, but encompasses any enzyme comprising an endoglucanase catalytic domain, therefore having endoglucanase activity, as previously defined

The term "endoglucanase catalytic domain", as used herein, refers to a domain of an enzyme being responsible of its catalytic function, particularly of its endoglucanase function. It contains the active site, a set of amino acids with a special spatial arrangement that permits interaction with the substrate to effect the reaction.

In the combination of the invention, the endoglucanase catalytic domain comprises or consists on a sequence selected from the group consisting of SEQ ID NO: 1, 2, 3, 4 and a functionally equivalent variant thereof.

Table 1. Sequences of the endoglucanase catalytic domains

| SEQ ID NO | OTHER DENOMINATION | SEQUENCE |
|---|---|---|
| 1 | LFCA | TPVETHGQLSVKGGQLVDENGKPVQLRGMSSHGLQ WFGDFVNKDSMKWLRDDWGINVFRVAMYTAEGGYI TNPSVKNKVKEAVEAAIDLGMYVIIDWHILSDNDPNT YKEQAKAFFQEMAAKYGNYPNVIYEICNEPNGGVTW SNQIKPYAEEVIPAIRANDPDNIIIVGTPTWSQDVHDA ADNPLPYSNIMYALHFYAGTHGQSLRDKIDYALSKGV AIFVTEWGTSDASGNGGPFLNESQKWIDFMNSRNIS WANWSLSDKSETSAALMPGASPTGGWTDSNLSAS GKFVREQIR |
| 2 | LCCA | TPVETHGQLSVKGGQLVDENGQPVQLRGMSSHGLQ WYGDFVNKDSMKWLRDDWGINVFRVAMYTAEGGYI TNPSVKNKVKEAVEAAIDLGLYVIIDWHILSDGDPNTY KEQAKAFFQEMATLYGNYPNVIYEICNEPNGNVTWS NQIKPYAEEVIPVIRANDPDNIIIVGTPTWSQDVHDAA DNPLPYSNIMYALHFYAGTHGQSLRDKIDYALNKGAA IFVTEWGTSDASGNGGPFLNESQEWIDFMNSRNISW ANWSLSDKSETSAALMPGASPTGGWTDSQLSASGK FVREQIR |
| 3 | LACA | TPVEINGQLQVCGVQLCNQYGKPIQLRGMSTHGIQW FGNCVNNASLDALANDWRADIFRIAMYIQEDGYETN PAGTNRVNNLVEEATARGMYVLIDWHILTPGDPNYN LDRAKTFFAEIAARHASKTNVIYEIANEPNGGVSWSN TIKSYAEEVIPVIRANDPDSVVIVGTRGWSSDSTEIVN NPVNASNIMYAFHFYAASHRDNYRDEVERAAARGLP VFVTEFGTVTYTGDGGNDLASSQKWLDLLDARKIGW ANWNFSDKAESSAALRPGTCAGGSWTGTSLTPSGV FVRERIR |

(continued)

| SEQ ID NO | OTHER DENOMINATION | SEQUENCE |
|---|---|---|
| 4 | | TPVETHGQLSVKGGQLVDENGKPVQLRGMSSHGLQ WFGDFVNKDSMKWLRDDWGINVFRVAMYTAEDGYI TNPSVKNKVKEAVEAAIDLGMYVIIDWHILSDNDPNT YKAQAKAFFQEMAALYGNYPNVIYEIANEPNGNVTW NNQIKPYAEEVIPVIRAKDPDNIIIVGTGTWSQDVHDA ADNPLPDSNIMYALHFYAGTHGQFLRDRIDYALSKGA AIFVTEWGTSDASGNGGPFLPESQEWIDFMNSRKIS WANWSLSDKSETSAALMPGASPTGGWTESQLSAS GKFVREQIR |

In a particular embodiment, the endoglucanase catalytic domain comprises or consists on the sequence of SEQ ID NO: 1. An enzyme comprising the endoglucanase catalytic domain of SEQ ID NO: 1 was described and characterized on international patent application WO2017/121902 and on Barruetabeña *et al.* (supra).

In a particular embodiment, the endoglucanase catalytic domain comprises or consists on the sequence of SEQ ID NO: 2. An enzyme comprising the endoglucanase catalytic domain of SEQ ID NO: 2 was described and characterized on Barruetabeña *et* al. (supra).

In a particular embodiment, the endoglucanase catalytic domain comprises or consists on the sequence of SEQ ID NO: 3. An enzyme comprising the endoglucanase catalytic domain of SEQ ID NO: 3 was described and characterized on international patent application WO2017/121902 and on Barruetabeña *et al.* (supra).

In a particular embodiment, the endoglucanase catalytic domain comprises or consists on the sequence of SEQ ID NO: 4. An enzyme comprising the endoglucanase catalytic domain of SEQ ID NO: 4 was described and characterized on international patent application WO2017/121902.

In a particular embodiment, the endoglucanase catalytic domain comprises or consists on a functionally equivalent variant of the sequences of SEQ ID NO: 1, 2, 3, or 4, preferably of SEQ ID NO: 1.

The term "functionally equivalent variant" as used herein is understood to mean all those proteins derived from a sequence by modification, insertion and/or deletion or one or more amino acids, whenever the function is substantially maintained, particularly in the case of a functionally equivalent variant of an endoglucanase catalytic domain refers to maintaining the endoglucanase catalytic activity.

A functionally equivalent variant of the catalytic domain of sequence SEQ ID NO: 1, 2, 3 or 4 can be an amino acid sequence derived from SEQ ID NO: 1, 2, 3 or 4 comprising the addition, substitution or modification of one or more amino acid residues. By way of illustration, functionally equivalent variants of the catalytic domain of sequence SEQ ID NO: 1, 2, 3 or 4 include sequences comprising the addition of 1 amino acid, 2 amino acids, 3 amino acids, 4 amino acids, 5 amino acids, 10 amino acids, 15 amino acids, 20 amino acids, 25 amino acids, 30 amino acids, 35 amino acids, 40 amino acids, 45 amino acids, 50 amino acids, 60 amino acids, 70 amino acids, 80 amino acids, 90 amino acids, 100 amino acids, 150 amino acids, 200 amino acids, at least 500 amino acids, at least 1000 amino acids or more at the amino terminus of the sequence SEQ ID NO: 1, 2, 3 or 4, and/or comprising the addition of 1 amino acid, 2 amino acids, 3 amino acids, 4 amino acids, 5 amino acids, 10 amino acids, 15 amino acids, 20 amino acids, 25 amino acids, 30 amino acids, 35 amino acids, 40 amino acids, 45 amino acids, 50 amino acids, 60 amino acids, 70 amino acids, 80 amino acids, 90 amino acids, 100 amino acids, 150 amino acids, 200 amino acids, at least 500 amino acids, at least 1000 amino acids or more at the carboxyl terminus of the sequence SEQ ID NO: 1, 2, 3 or 4.

Functionally equivalent variants of a catalytic domain of sequence SEQ ID NO: 1, 2, 3 or 4 also include sequences with a sequence identity of at least 70%, at least 75%, at least 80%, at least 85%, at least 90%, at least 91%, at least 92%, at least 93%, at least 94%, at least 95%, at least 96%, at least 97%, at least 98%, or at least 99% with the sequences of SEQ ID NO: 1, 2, 3 or 4

The terms "identity", "identical" or "percent identity" in the context of two or more amino acid sequences, refer to two or more sequences or subsequences that are the same or have a specified percentage of amino acid residues that are the same, when compared and aligned (introducing gaps, if necessary) for maximum correspondence, not considering any conservative amino acid substitutions as part of the sequence identity. The percent identity can be measured using sequence comparison software or algorithms or by visual inspection. Various algorithms and software are known in the art that can be used to obtain alignments of amino acid or nucleotide sequences. One such non-limiting example of a sequence alignment algorithm is the algorithm described in Karlin et al., 1990, Proc. Natl. Acad. Sci., 87:2264-8, as modified in Karlin et al., 1993, Proc. Natl. Acad. Sci., 90:5873-7, and incorporated into the NBLAST and XBLAST programs (Altschul et al., 1991, Nucleic Acids Res., 25:3389-402). In certain embodiments, Gapped BLAST can be used as described in Altschul et al., 1997, Nucleic Acids Res. 25:3389-402. BLAST-2, WU-BLAST-2 (Altschul et al., 1996, Methods in Enzymology, 266:460-80), ALIGN, ALIGN-2 (Genentech, South San Francisco, California) or Megalign (DNASTAR) are additional publicly available software programs that can be used to align sequences. In certain embodiments, the percent identity between two nucleotide sequences is determined using the GAP program in GCG software (e.g., using a NWSgapdna.CMP matrix and a gap weight of 40, 50, 60, 70, or 90 and a length weight of 1, 2, 3, 4, 5, or 6). In certain alternative embodiments, the GAP program in the GCG software package, which incorporates the algorithm of Needleman and Wunsch (J. Mol. Biol. 48:444-53 (1970)) can be used to determine the percent identity between two amino acid sequences (e.g., using either a Blossum 62 matrix or a PAM250 matrix, and a gap weight of 16, 14, 12, 10, 8, 6, or 4 and a length weight of 1, 2, 3, 4, 5). Alternatively, in certain embodiments, the percent identity between nucleotide or amino acid sequences is determined using the algorithm of Myers and Miller (CABIOS, 4:11-7 (1989)). For example, the percent identity can be determined using the ALIGN program (version 2.0) and using a PAM120 with residue table, a gap length penalty of 12 and a gap penalty of 4. Appropriate parameters for maximal alignment by particular alignment software can be determined by one skilled in the art. In certain embodiments, the default parameters of the alignment software are used.

In certain embodiments, the percentage identity "X" of a first amino acid sequence to a second sequence amino acid is calculated as 100 x (Y/Z), where Y is the number of amino acid residues scored as identical matches in the alignment of the first and second sequences (as aligned by visual inspection or a particular sequence alignment program) and Z is the total number of residues in the second sequence. If the second sequence is longer than the first sequence, then the percent identity may be determined only in the region of overlap between said first and second sequences. In this case, the same formula as above can be used but using as Z value the length of the region wherein the first and second sequence overlaps, said region having a length which is substantially the same as the length of the first sequence.

In a particular embodiment, the sequence identity is determined throughout the whole length of the sequence of the endoglucanase domain comprising the sequence of SEQ ID NO: 1, 2, 3 or 4, or through the whole length of the variant or both.

As a non-limiting example, whether any particular polynucleotide has a certain percentage sequence identity (e.g., is at least 70% identical, at least 75% identical, at least 80% identical, at least 85% identical, at least 90% identical, and in some embodiments, at least 95%, 96%, 97%, 98%, or 99% identical) to a reference sequence can, in certain embodiments, be determined using the Bestfit program (Wisconsin Sequence Analysis Package, Version 8 for Unix, Genetics Computer Group, University Research Park, 575 Science Drive, Madison, WI 53711). Bestfit uses the local homology algorithm of Smith and Waterman, Advances in Applied Mathematics 2:482-9 (1981), to find the best segment of homology between two sequences. When using Bestfit or any other sequence alignment program to determine whether a particular sequence is, for instance, 95% identical to a reference sequence according to the present invention, the parameters are set such that the percentage of identity is calculated over the full length of the reference amino acid sequence and that gaps in homology of up to 5% of the total number of nucleotides in the reference sequence are allowed.

In some embodiments, two amino acid sequences are substantially identical, meaning they have at least 70%, at least 75%, at least 80%, at least 85%, at least 90%, and in some embodiments at least 95%, 96%, 97%, 98%, 99% amino acid residue identity, when compared and aligned for maximum correspondence, as measured using a sequence comparison algorithm or by visual inspection. Identity can exist over a region of the sequences that is at least about 10, about 20, about 40-60 residues in length or any integral value there between, and can be over a longer region than 60-80 residues, for example, at least about 90-100 residues, and in some embodiments, the sequences are substantially identical over the full length of the sequences being compared.

In a particular embodiment, suitable functionally equivalent variants of endoglucanase catalytic domain of SEQ ID NO: 1, 2, 3 or 4 are those including one or more conservative substitutions of the amino acids. "Conservative amino acid substitutions" result from replacing one amino acid with another having similar structural and/or chemical properties For example, the following six groups each contain amino acids that are conservative substitutions for one another: 1) Alanine (A), Serine (S), Threonine (T); 2) Aspartic acid (D), Glutamic acid (E); 3) Asparagine (N), Glutamine (Q); 4) Arginine (R), Lysine (K); 5) Isoleucine (I), Leucine (L), Methionine (M), Valine (V); and 6) Phenylalanine (F), Tyrosine (Y), Tryptophan (W). Selection of such conservative amino acid substitutions is within the skill of one of ordinary skill in the art and is described, for example by Dordo et al. et al., [J. Mol. Biol, 1999, 217;721-739] and Taylor et al., [J. Theor. Biol., 1986, 119:205-218].

In a particular embodiment, the functionally equivalent variant of the endoglucanase catalytic domain of SEQ ID NO: 1 does not comprise a deletion or substitution of the following amino acid residues of SEQ ID NO: 1: E136, E224, W174 and W258.

A functionally equivalent variant of the endoglucanase catalytic domain of sequence SEQ ID NO: 1, 2, 3 or 4 substantially maintains its endoglucanase catalytic activity.

The term "endoglucanase catalytic activity", as used herein, refers to the ability of hydrolysing 1,4-linkages in $\beta$-D-glucans also containing 1,3-linkages. Assays to determine the function of an enzyme are known by the skilled person and include, without limitation, initial rate assays, progress curve assays, transient kinetics assays and relaxation assays.

Continuous assays of enzymatic activity include, without limitation, spectrophotometric, fluorometric, calorimetric, chemiluminiscent, light scattering and microscale thermopheresis assays. Discontinuous assays of enzymatic activity include, without limitation, radiometric and chromatographic assays. As the skilled person understands, factors that may influence enzymatic activity comprise salt concentration, temperature, pH, and substrate concentration. Particularly, the endoglucanase catalytic activity may be measured by means of a number of techniques assays that are conventional to the skilled person, including the viscosimetric methods using soluble derivatised cellulose, or by employing soluble or insoluble (crosslinked) dyed cellulose or mixed-linkage β-glucan, such as the carboxymethyl cellulose (CMC) assay, and the hydroxyethylcellulose (HEC) assay. In general, assays based on the use of dyed polysaccharides are standardised against a reducing sugar method that employs either CM-cellulose or β-glucan as substrate. In a particular embodiment, the CELLG3 assay (particularly K-CellG3, Megazyme International Ireland) may be used for specific endocellulases and measures activity.

As with other enzymes, the catalytic activity of the endoglucanase catalytic domain depends on a number of reaction parameters, including temperature and pH. Thus, in one embodiment, the functionally equivalent variant of the endoglucanase catalytic domain of sequence SEQ ID NO: 1, 2, 3 or 4 maintains its endoglucanase catalytic activity at a temperature of at least 0°C, at least 5°C, at least 10°C, at least 15°C, at least 20°C, at least 25°C, at least 30°C, at least 35°C, at least 37°C, at least 40°C, at least 45°C, at least 50°C, at least 55°C, at least 60°C, at least 65°C, at least 70°C, at least 75°C, at least 80°C, at least 85°C, at least 90°C, at least 95°C, at least 100°C, or higher. Likewise, in another embodiment the functionally equivalent variant of the endoglucanase catalytic domain of sequence SEQ ID NO: 1, 2, 3 or 4 maintains its endoglucanase catalytic activity at pH 0, or at least pH 0.1, or at least pH 0.5, or at least pH 1.0, or at least pH 1.5, or at least pH 2.0, or at least pH 2.5, or at least pH 3.0, or at least pH 3.5, or at least pH 4.0, or at least pH 4.5, or at least pH 5.0, or at least pH 5.5, or at least pH 6.0, or at least pH 6.5, or at least pH 7.0, or at least pH 7.5, or at least pH 8.0, or at least pH 8.5, or at least pH 9.0, or at least pH 9.5, or at least pH 10.0, or at least pH 10.5, or at least pH 11.0, or at least pH 11.5, or at least pH 12.0, or at least pH 12.5, or at least pH 13.0, or at least pH 13.5, or pH 14. All possible combinations of temperatures and pH are also contemplated by the invention.

In a particular embodiment, the functionally equivalent variant of the endoglucanase catalytic domain of SEQ ID NO: 1, 2, 3 or 4 maintains at least 50%, at least 60%, at least 70%, at least 75%, at least 0%, at least 85%, at least 90%, at least 91%, at least 92%, at least 93%, at least 94%, at least 95%, at least 96%, at least 97%, at least 98%, at least 99%, or at least 100% of the endoglucanase catalytic activity of the catalytic domain of sequence SEQ ID NO: 1, 2, 3 or 4. In the context of the combination of the invention, the expression "maintains its endoglucanase activity" encompass also an improvement it the endoglucanase activity, for example, improving it in at least 1%, at least 2%, at least 3%, at least 4%, at least 5%, at least 6%, at least 7%, at least 8%, at least 9%, at least 10%, at least 15%, at least 20%, at least 25%, at least 30%, at least 35%, at least 40%, at least 45%, at least 50%, at least 55%, at least 60%, or at least 65%, at least 70%, at least 75%, at least 80%, at least 85%, at least 90%, at least 95%, at least 100%, at least 150%, at least 200%, at least 300%, at least 400%, at least 500%, at least 1000%, or more.

In a particular embodiment, the functionally equivalent variant of the endoglucanase catalytic domain of SEQ ID NO: 1, 2, 3 or 4 comprises or consist of an amino acid sequence with a sequence identity of at least 70%, at least 75%, at least 80%, at least 85%, at least 90%, at least 91%, at least 92%, at least 93%, at least 94%, at least 95%, at least 96%, at least 97%, at least 98%, or at least 99% with the sequences of SEQ ID NO: 1, 2, 3 or 4, and maintains at least 50%, at least 60%, at least 70%, at least 75%, at least 80%, at least 85%, at least 90%, at least 91%, at least 92%, at least 93%, at least 94%, at least 95%, at least 96%, at least 97%, at least 98%, at least 99%, or at least 100% of the endoglucanase catalytic activity of the endoglucanase catalytic domain of sequence SEQ ID NO: 1, 2, 3 or 4, or improves it in at least 1%, at least 2%, at least 3%, at least 4%, at least 5%, at least 6%, at least 7%, at least 8%, at least 9%, at least 10%, at least 15%, at least 20%, at least 25%, at least 30%, at least 35%, at least 40%, at least 45%, at least 50%, at least 55%, at least 60%, or at least 65%, at least 70%, at least 75%, at least 80%, at least 85%, at least 90%, at least 95%, at least 100%, at least 150%, at least 200%, at least 300%, at least 400%, at least 500%, at least 1000%, or more.

In a particular embodiment, the functionally equivalent variant of the endoglucanase catalytic domain of SEQ ID NO: 1, 2, 3 or 4 comprises or consist of an amino acid sequence with a sequence identity of at least 70% with the sequences of SEQ ID NO: 1, 2, 3 or 4, preferably SEQ ID NO: 1, and maintains at least 50% of the endoglucanase catalytic activity of the endoglucanase catalytic domain of sequence SEQ ID NO: 1, 2, 3 or 4, preferably SEQ ID NO: 1.

In a particular embodiment, the functionally equivalent variant of the endoglucanase catalytic domain of sequence SEQ ID NO: 1, 2, 3 or 4 is not the catalytic domain of an endoglucanase selected from the group consisting of:

- The endocellulase shown in the UniProt database with Accession No. A0A0B2T1H3,
- The endocellulase shown in the UniProt database with Accession No. U3RD83,
- The endocellulase shown in the EMBL database with Accession No. KF240855,
- The endocellulase shown in the UniProt database with Accession No. U3R8L0,
- The endocellulase shown in the EMBL database with Accession KF240853

- The endocellulase shown in the GenSeq database with accession number BCD39860.
- The endocellulase shown in international publication WO2008005529 under SEQ ID NO:60,
- The endocellulase shown in international publication WO2008005529 under SEQ ID NO:59,
- The plant biomass degrading enzyme #67 shown in international publication WO2009208941,
- The endocellulase shown in international publication WO2011109905 under SEQ ID NO:15,
- The endocellulase shown in the UniProt database with Accession No. A0B0B2T1H3,
- The endocellulase shown in the UniProt database with Accession No. F4FAV2,
- The endocellulase shown in the UniProt database with Accession No. A0A0D0X703
- The endocellulase shown in the UniProt database with Accession No P26224.

In a particular embodiment, the enzyme comprising an endoglucanase catalytic domain further comprises a carbohydrate binding domain.

The term "carbohydrate binding domain" or "carbohydrate binding module" or "CBM", as used herein, refers to a protein domain that is present in carbohydrate-active enzymes (for example endocellulases and exocellulases) and having carbohydrate-binding activity. Carbohydrate binding domains contributes to the catalytic efficiency by increasing enzyme-substrate complex formations.

In a particular embodiment, the CBM comprises, or consists of a sequence selected from the group consisting of SEQ ID NO: 6 to 35.

The particulars of a functionally equivalent variant in terms of sequence identity previously described in the context of the endoglucanase catalytic domain also apply to the carbohydrate binding domain, with the necessary amendments, as will be evident for the person skilled in the art.

Thus, functionally equivalent variants of a carbohydrate binding domain of sequence SEQ ID NO: 6 to 35 also include carbohydrate binding domains comprising or consisting of amino acid sequences with a sequence identity of at least 70%, at least 75%, at least 80%, at least 85%, at least 90%, at least 91%, at least 92%, at least 93%, at least 94%, at least 95%, at least 96%, at least 97%, at least 98%, or at least 99% with the sequence SEQ ID NO: 6 to 35.

In a particular embodiment, the functionally equivalent variant of the carbohydrate binding domain of SEQ ID NO: 6 to 35 maintains at least 50%, at least 60%, at least 70%, at least 75%, at least 80%, at least 85%, at least 90%, at least 91%, at least 92%, at least 93%, at least 94%, at least 95%, at least 96%, at least 97%, at least 98%, at least 99%, or at least 100% of its carbohydrate binding activity or improves it in at least 1%, at least 2%, at least 3%, at least 4%, at least 5%, at least 6%, at least 7%, at least 8%, at least 9%, at least 10%, at least 15%, at least 20%, at least 25%, at least 30%, at least 35%, at least 40%, at least 45%, at least 50%, at least 55%, at least 60%, or at least 65%, at least 70%, at least 75%, at least 80%, at least 85%, at least 90%, at least 95%, at least 100%, at least 150%, at least 200%, at least 300%, at least 400%, at least 500%, at least 1000%, or more.

The activity of a carbohydrate binding domain may be measured as the affinity of said domain for cellulose, for example using biotinylated glycan binding assay or enzyme-linked assay (Kim et al., Biotecnhology and bioprocess engineering 19: 575-580 (2013).

In a particular embodiment, the endoglucanase catalytic domain and the carbohydrate binding domain are connected by a linking domain.

The term "linking domain", as used herein, refers to a sequence between domains. Linkers are often composed of flexible residues like glycine and serine so that the adjacent protein domains are free to move relative to one another. Longer linkers are used when it is necessary to ensure that two adjacent domains do not sterically interfere with one another.

In a particular embodiment, the linking domain comprises or consist of the sequence of SEQ ID NO: 35 or a variant thereof, said linking domain being located between the catalytic domain and the carbohydrate binding domain.

Functionally equivalent variants of a linking domain of sequence SEQ ID NO: 36 also include amino acid sequences with a sequence identity of at least 70%, at least 75%, at least 80%, at least 85%, at least 90%, at least 91%, at least 92%, at least 93%, at least 94%, at least 95%, at least 96%, at least 97%, at least 98%, or at least 99% with the sequence of SEQ ID NO: 36.

In a particular embodiment, the variant of the linking domain is a functionally equivalent variant thereof. The particulars of a functionally equivalent variant previously described in the context of the endoglucanase catalytic domain also apply to the linking domain, with the necessary amendments, as will be evident for the person skilled in the art.

Component (ii) of the combination of the invention is a loosenin or a loosenin-like protein.

The term "loosenin", as used herein, refers to expansin-related proteins comprising only the D1 domain. Expansins are non-lytic proteins comprising an N-terminal six-stranded double-Ψ beta-barrel (DPBB) domain (D1) connected via a short-linker to a C-terminal domain (D2). Expansins and expansin related proteins are believed to disrupt noncovalent bonds between cellulose microfibrils and matrix polysaccharides. Loosenins, which as mentioned above only include the D1 domain, can enhance the enzymatic breakdown of lignocellulosic substrates. Examples of loosenins include LOOS1 from *Bjerkandera adusta* (UniprotKB accession number D8WUM3, entry version 26, sequence version 2, 24 July 2024) and N2 from *Neurospora crassa* (Campos-Oliver, A., Quiroz-Castañeda, R., Ortiz-Suri, E. et al. Cloning and expression of a hypothetical Loosenin from Neurospora crassa. Rev Latinoam Biotecnol Ambient Algal 5, 1 (2014). https://doi.org/10.7603/s40682-014-0001-y; NCBI Reference sequence: XP_959591.1).

The term "loosenin-like protein" or "LOOL" a used herein, refers to proteins homologous to known loosenins, particularly to LOOS1. Illustrative non-limitative examples of LOOL includes the 12 LOOL proteins described by Suzuki H, et al., Fungal Genet Biol. 2014 Nov;72:115-123.

In a particular embodiment, the LOOL protein is any of the LOOL proteins from *P. carnose* listed below:

- LOOL1, GenBank accession number: EKM57382.
- LOOL2, GenBank accession number: EKM55931.
- LOOL3, GenBank accession number: EKM55664.
- LOOL4, GenBank accession number: EKM55665.
- LOOL5, GenBank accession number: EKM54243.
- LOOL6, GenBank accession number: EKM53476.
- LOOL7, GenBank accession number: EKM53490.
- LOOL8, GenBank accession number: EKM52556.
- LOOL9, GenBank accession number: EKM52742.
- LOOL10, GenBank accession number: EKM52733.
- LOOL11, GenBank accession number: EKM52777.
- LOOL12, GenBank accession number: EKM51974, with the sequence of SEQ ID NO: 5. The terms "PcaLOOL12", "LOOL12" and "loosenin-like protein of SEQ ID NO: 5" are used herein interchangeably.

In an even more particular embodiment, wherein the loosenin-like protein is the loosenin-like protein from *Phanerochaete carnosa* having the sequence of SEQ ID NO: 5 or a functionally equivalent variant thereof.

The particulars of a functionally equivalent variant in terms of sequence identity previously described in the context of the endoglucanase catalytic domain also apply to the loosenin-like protein, with the necessary amendments, as will be evident for the person skilled in the art.

Thus, functionally equivalent variants of a loosenin-like protein of SEQ ID NO: 5 also include variants comprising or consisting of amino acid sequences with a sequence identity of at least 70%, at least 75%, at least 80%, at least 85%, at least 90%, at least 91%, at least 92%, at least 93%, at least 94%, at least 95%, at least 96%, at least 97%, at least 98%, or at least 99% with the sequence SEQ ID NO: 5.

In a particular embodiment, the functionally equivalent variant of the loosenin-like protein of SEQ ID NO: 5 maintains at least 50%, at least 60%, at least 70%, at least 75%, at least 80%, at least 85%, at least 90%, at least 91%, at least 92%, at least 93%, at least 94%, at least 95%, at least 96%, at least 97%, at least 98%, at least 99%, or at least 100% of its activity or improves it in at least 1%, at least 2%, at least 3%, at least 4%, at least 5%, at least 6%, at least 7%, at least 8%, at least 9%, at least 10%, at least 15%, at least 20%, at least 25%, at least 30%, at least 35%, at least 40%, at least 45%, at least 50%, at least 55%, at least 60%, or at least 65%, at least 70%, at least 75%, at least 80%, at least 85%, at least 90%, at least 95%, at least 100%, at least 150%, at least 200%, at least 300%, at least 400%, at least 500%, at least 1000%, or more.

LOOL12 has been shown to boost or increase the enzymatic conversion of a lignocellulosic substrate by cellulases (Deepika Dahiya, et al., Bioresource Technology, Volume 394, 2024, 130188.). Therefore, in an embodiment, the functionally equivalent variants of the protein of SEQ ID NO: 5 substantially maintains its ability to increase the enzymatic degradation of a lignocellulosic substrate by cellulases. The ability to increase, boost or potentiate the enzymatic degradation of a lignocellulosic substrate by a cellulase can be measured by any method that can determine the degradation of a lignocellulosic substrate (for example, measuring total reducing sugars or monosaccharide release) by a cellulase, for example an endoglucanase, in the presence and absence of LOOL12 or a variant thereof. Such methods are for example described in Deepika Dahiya et al. (supra).

As shown in the Examples herein included, LOOL12 is capable of boosting, improving or increasing crystalline nanocellulose (CNC) production from a cellulosic substrate (in particular filter paper) by an enzyme comprising an endoglucanase domain of SEQ ID NO: 1. Therefore, in another embodiment, the functionally equivalent variants of the protein of SEQ ID NO: 5 substantially maintains its ability to boost, improve or increase CNC production from a cellulosic substrate by an enzyme comprising an endoglucanase domain of SEQ ID NO: 1. The ability to boost, improve or increase CNC production from a cellulosic substrate by an enzyme comprising the endoglucanase domain of SEQ ID NO: 5 can be determined by any method that quantifies CNC production, such as the methods disclosed in the Examples herein included. In a more particular embodiment, the effect (boost, improve or increase) on CNC production is an increase in the CNC yield. In another particular embodiment, the effect (boost, improve or increase) on CNC production in a decrease in the time needed to complete the reaction. In another particular embodiment, the effect (boost, improve or increase) on CNC production is an improved in the aspect ratio of the CNC (CNC with lower aspect ratio). In another embodiment, the effect (boost, improve or increase) on CNC production is a reduction of the length of the CNC.

In a particular embodiment, the combination comprises an enzyme comprising an endoglucanase catalytic domain, wherein the endoglucanase catalytic domain comprises the sequence of SEQ ID NO: 1, and a loosenin-like protein, wherein the loosenin-like protein comprises the sequence of SEQ ID NO: 4.

In a particular embodiment, components (i) and (ii) are included in the same formulation. In a particular embodiment, components (i) and (ii) are included in separate formulations.

_Method for producing crystalline nanocellulose_

In another aspect, the invention relates with a method for producing crystalline nanocellulose comprising contacting a substrate comprising cellulose with the combination of the invention under suitable conditions for hydrolyzing cellulose.

The combination of the invention has been previously defined. All the particular and preferred embodiments of the combination of the invention also apply to the method for producing crystalline nanocellulose of the invention.

The term "crystalline nanocellulose" or "cellulose nanocrystals" or "CNC" or "nanowhiskers", as used herein, refers to a still rod-like nanoparticles consisting of cellulose chain segments with very high crystallinity. CNC have normally an average diameter between 2-30 nm and a length of 10 nm to 2 $\mu$m, depending on the cellulose source and the isolation treatment, as opposed to cellulose nanofibers (CNF), which are long thin fibers with a length of several micrometers and diameters of nanometers, and include both amorphous and crystalline domains. Another difference between CNC and CNF is the aspect ratio (length/diameter). It is generally accepted that CNF have an aspect ratio over 100, while the aspect ratio of CNC is generally lower.

The method of the invention comprises contacting a substrate comprising cellulose with the combination of the invention.

The term "cellulose" as used herein, refers to an organic compound with CAS number 9004-34-6, a polysaccharide consisting of a linear chain of several hundred to many thousands of $\beta(1\rightarrow4)$ linked D-glucose units. Cellulose is a semi-crystalline polymer comprising crystalline and amorphous regions. Crystalline domains are very packed together and are very difficult to degrade by either chemical or enzymatic treatment. The amorphous regions are easier to degrade. The proportion between the amorphous and the crystalline regions depends on the cellulose source and on the treatment used to extract the fibers. There are six polymorphs described in the literature: cellulose I, II, III$_I$, III$_{II}$, IV$_I$ and IV$_{II}$. The native and most abundant form of cellulose is type I, and all polymorphs can be produced from it with different physicochemical treatments. Cellulose I has the chains of the polymer in a parallel organization and it can also be organized in two polymorphs, cellulose I$\alpha$ and I$\beta$. These two type I polymorphs can be found together, and the ratio between them vary with the cellulose source. Cellulose I$\alpha$ is abundant in algae and bacterial cellulose and cellulose I$\beta$ is more present in higher plants and tunicates. Cellulose II is the second most abundant cellulose form; in this polymorph, cellulose chains have an antiparallel organization. This distribution permits that hydrogen bonds happen between the neighbor's hydroxyl groups and improved the interlayer attraction forces, but there are less secondary hydrogen bonds. These interactions made that cellulose II has added stability and produce the irreversibility to convert cellulose II into I. Also, cellulose II is more thermostable and weaker mechanically than cellulose I because of its different chain organization. Cellulose II can be prepared from cellulose I by mercerization (alkaline treatment) or regeneration. From cellulose I and II the other polymorphs can be produced. Cellulose III is made from cellulose I or cellulose II by ammonia treatment that penetrates and degrades the cellulose crystal structure. Cellulose IV is produced from cellulose III by glycerol and heat treatment at 260 °C. Cellulose III can be transformed into their previous polymorphs by alkaline treatment.

In a particular embodiment, the substrate comprising cellulose comprises amorphous and crystalline regions. In a particular embodiment, the substrate comprises type I cellulose.

The term "substrate comprising cellulose", as use herein, refers to any material comprising cellulose, preferably type I cellulose. Illustrative non-limitative examples of said substrates are lignocellulose biomass (composed mainly of cellulose, hemicellulose and lignin), corn stover, _Panicum virgatum_ (switchgrass), Miscanthus grass species, wood chips and the byproducts of lawn and tree maintenance. Lignocellulosic biomass can be grouped into four main categories: (1) agricultural residues (including corn stover and sugarcane bagasse), (2) dedicated energy crops, (3) wood residues (including sawmill and paper mill discards), and (4) municipal paper waste. Illustrative lignocellulosic biomass sources include, but are not limited to grasses, rice hulls, bagasse, jute, hemp, flax, bamboo, sisal, abaca, straw, corn cobs, corn stover, alfalfa, hay, coconut hair, seaweed and algae.

In a particular embodiment, the substrate comprising cellulose comprises or consists on pure cellulose. The term "pure cellulose", as used herein, refers to cellulose that has generally been separated from other plant natural products, such as hemicellulose and/or lignin and typically has a purity of 90% w/w or more, e.g. 95% w/w or more. Examples of pure cellulose substrates are filter paper or cellulose threads. In a more particular embodiment, the substrate comprising or consisting of pure cellulose is filter paper.

In a particular embodiment, the substrate comprising cellulose does not comprises pure cellulose. In a more particular embodiment, the substrate comprising cellulose is a lignocellulosic substrate. The term "lignocellulosic substrate", as used herein, refers to a material, usually derived from plant biomass, which comprises cellulose, hemicellulose and lignin. The lignocellulosic material can be derived from a single material or a combination of materials and/or can be non-modified and/or modified. Lignocellulosic material can be transgenic (i.e., genetically modified). Lignocellulose is generally found, for example, in the fibers, pulp, stems, leaves, hulls, canes, husks, and/or cobs of plants or fibers, leaves, branches, bark, and/or wood of trees and/or bushes. Examples of lignocellulosic materials include, but are not limited to, agricultural biomass, e.g., farming and/or forestry material and/or residues, branches, bushes, canes, forests, grains, grasses, short rotation woody crops, herbaceous crops, and/or leaves; oil palm fiber waste such as empty fruit bunch and palm trunk; energy crops, e.g., corn, millet, and/or soybeans; energy crop residues; paper mill residues; sawmill residues; municipal paper waste; orchard prunings; Willow coppice and Mallee coppice; wood waste; wood chip, logging waste; forest thinning; short-rotation woody crops; bagasse, such as sugar cane bagasse and/or sorghum bagasse, duckweed; wheat straw; oat straw; rice straw; barley straw; rye straw; flax straw; soy hulls; rice hulls; rice straw; tobacco; corn gluten feed; oat hulls; corn kernel; fiber from kernels; corn stover; corn stalks; com cobs; corn husks; canola; miscanthus; energy cane; prairie grass; gamagrass; foxtail; sugar beet pulp; citrus fruit pulp; seed hulls; lawn clippings; cotton, seaweed; trees; shrubs; wheat; wheat straw; products and/or by-products from wet or dry milling of grains; yard waste; plant and/or tree waste products; herbaceous material and/or crops; forests; fruits; flowers; needles; logs; roots; saplings; shrubs; switch grasses; vegetables; fruit peels; vines; wheat midlings; oat hulls; hard and soft woods; or any combination thereof. In another embodiment, the lignocellulosic material may be the product obtained by a processor selected from the group consisting of a dry grind ethanol production facility, a paper pulping facility, a tree harvesting operation, a sugar cane factory, or any combination thereof.

In a particular embodiment, the lignocellulosic material is paper pulp.

The term "paper pulp" means any pulp which can be used for the production of a paper material. For example, the pulp can be supplied as a virgin pulp, or can be derived from a recycled source. The pulp may be a wood pulp, a non-wood pulp or a pulp made from waste paper. A wood pulp may be made from softwood such as pine, redwood, fir, spruce, cedar and hemlock or from hardwood such as maple, alder, birch, hickory, beech, aspen, acacia and eucalyptus. A non-wood pulp may be made, e.g., from flax, hemp, bagasse, bamboo, cotton or kenaf. A waste paper pulp may be made by repulping waste paper such as newspaper, mixed office waste, computer print-out, white ledger, magazines, milk cartons, paper cups etc. The wood pulp may be mechanical pulp (such as ground wood pulp, GP), chemical pulp (such as Kraft pulp or sulfite pulp), semichemical pulp (SCP), thermomechanical pulp (TMP), chemithermomechanical pulp (CTMP), or bleached chemithermomechanical pulp (BCTMP).

Mechanical pulp is manufactured by the grinding and refining methods, wherein the raw material is subjected to periodical pressure impulses. TMP is thermomechanical pulp, GW is groundwood pulp, PGW is pressurized groundwood pulp, RMP is refiner mechanical pulp; PRMP is pressurized refiner mechanical pulp and CTMP is chemithermimechanical pulp.

Chemical pulp is manufactured by alkaline cooking whereby most of the lignin and hemicellulose components are removed. In Kraft pulping or sulphate cooking sodium sulphide or sodium hydroxide are used as principal cooking chemicals.

In a particular embodiment, the lignocellulosic material is unbleached kraft pulp (KAPPA). KAPPA is a pulp rich in cellulose (about 70%) but also comprising hemicellulose (about 10%), lignin (about 12%) and other components as described by Da Silva, T.A. et al., BioResources 2007, 2(4): 616-629. In another particular embodiment, the lignocellulosic material is bleached kraft pulp (BKP). BKP comprises more cellulose (about 80%), hemicellulose (12%) and a small lignin residue (about 1%) as described by Hu, J. et al., Sci. Rep. 2018, 8(1): 3195.

The method of the invention comprises contacting the substrate comprising cellulose with the combination of the invention under suitable conditions for hydrolyzing cellulose.

The term "hydrolyzing cellulose", as used herein, refers to the cleavage of chemical bonds of cellulose. Therefore, the conditions suitable for hydrolyzing cellulose are those conditions, which can be easily determined by the skilled person, under which at least part of the (1-4)-β-D-glucosidic links in cellulose are cleaved.

Without wanting to be bound to any theory, it is understood that endoglucanases, and as such also the enzyme comprising an endoglucanase catalytic domain comprising the sequence of SEQ ID NO: 1, 2, 3 or 4, hydrolyse the amorphous regions of cellulose more easily than the crystalline domains. Therefore, depending on the conditions under which the substrate comprising cellulose and the enzyme are contacted, nanocelullose particles of different length, diameter, aspect/ratio and crystallinity can be obtained in different proportions.

In a particular embodiment, the suitable conditions comprise contacting the substrate comprising cellulose and the combination of the invention under a pH between 4 and 10, particularly, about 4.0, about 4.5, about 5.5, about 5.5, about 6.0, about 6.5, about 6.8, about 7.0, about 7.2, about 7.4, about 7.6, about 7.8, about 8.0, about 8.5, about 9.0, about 9.5, about 9.8, more particularly between approximately 4.5 and 6.5.

In a particular embodiment, the suitable conditions comprise contacting the substrate comprising cellulose and the combination of the invention under a temperature between 25°C and 80°C, between 30°C and 70°C, between 40°C and 60°C, between 45°C and 55°C, for example approximately 30°C, approximately 35°C, approximately 40°C, approximately 45°C, approximately 50°C, approximately 55°C, approximately 60°C, approximately 65°C, approximately 70°C, approximately 75°C, approximately 80°C, preferably approximately 40°C.

In a particular embodiment, the suitable conditions comprise contacting the substrate comprising cellulose and the combination of the invention for a period of time of less than 72 hours, preferably less than 48 hours, more preferably at least 24 hours. In a more particular embodiment, the substrate comprising cellulose and the enzyme are contacted for a period of time comprised between 1 and 48 hours, for example, 2 hours, 4, hours, 5 hours, 6 hours, 8 hours, 10 hours, 12 hours, 14 hours, 16 hours, 18 hours, 20 hours, 22 hours, 24 hours, 26 hours, 28 hours, 30 hours, 32 hours, 34 hours, 36 hours, 38 hours, 40 hours, 42 hours, 44 hours, 46 hours, or 48 hours.

In a particular embodiment, the suitable conditions for hydrolyzing cellulose comprise contacting the substrate and the combination at a temperature of approximately 40°C and a pH between approximately 4.5 to 6.5 during at least approximately 24 hours.

In a particular embodiment, the sample comprising cellulose is chopped into smaller pieces to accelerate the process of enzymatic hydrolysis.

In a particular embodiment, the substrate comprising cellulose and the combination are mixed together in water to form a suspension.

In a particular embodiment, the substrate comprising cellulose and the combination are contacted under agitation.

If the combination comprises components (i) and (ii) in a single formulation, both components are contacted with the substrate comprising cellulose at the same time.

If the combination comprises components (i) and (ii) in a separate formulations, the method can comprise contacting component (i) with the substrate comprising cellulose, and then adding component (ii) to the mixture; or contacting component (ii) with the substrate comprising cellulose, and then adding component (i) to the mixture; or contacting component (i) and (ii) with the substrate comprising cellulose at the same time.

Once the above period of time is completed, that is, once the desired crystalline nanocellulose is obtained, the hydrolysis reaction can be stopped, for example, by decreasing the temperature of the reaction mixture to a temperature at which no hydrolysis is performed, for example, by incubation on ice.

Once the above period of time is completed, that is, once the desired crystalline nanocellulose is obtained, the hydrolysis reaction can be subjected to sonication.

In a particular embodiment, the resulting crystalline nanocellulose can be isolated from the rest of the components of the reaction mixture, for example, substrate that has not been hydrolysed, enzyme, or other products of the reaction, such as sugars and nanocellulose fibers (CNF). In a more particular embodiment, the crystalline nanocellulose is isolated by filtration and/or centrifugation and/or any other suitable technique known by the skilled person. In a particular embodiment, the crystalline nanocellulose is isolated by filtration. In another particular embodiment, the crystalline nanocellulose is isolated by centrifugation. In another particular embodiment, the crystalline nanocellulose is isolated by filtration and centrifugation.

The filtration can be performed by using microfiltration, a rotatory drum filter or using a nylon filter of 5 to 20 $\mu$m mesh. To reach the maximum recovery of cellulose nanocrystals, the retentate can be resuspended in a lower volume of water and washed several times, repeating the filtration until the filtrate is clear. The cellulose nanocrystals can be recovered from the filtrate and washings by centrifugation at 15000 g (relative centrifuge force) for 30 minutes to 1 hour.

The isolation by centrifugation can be performed in two steps, a first step to wash the undigested fibers, recovering the crystalline nanocellulose from the supernatant of successive washings with decreasing volumes of water followed by centrifugation at 4000 g (relative centrifuge force) for 5 minutes. The washings should be repeated suspending the sediment in water until the supernatant is clear and free of nanocellulose material. The second recovery step concentrates the suspended material of the supernatant of previous washings by higher speed centrifugation at 15000 g for 30 minutes to 1 hour. After centrifugation, the sediment comprising the crystalline nanocellulose can be dried, for example by spray drying or lyophilisation.

The method of the invention can optionally comprise one or more mechanical treatments to aid in the hydrolysis of the substrate by the enzyme. However, in a particular embodiment, the method does not comprise a step of microfluidization, microwaving, wet disk milling, mechanical shearing and/or bleaching.

The term "microfluidization", as used herein, refers to a process of physical disruption via two high pressure streams of liquid at high velocities.

The term "microwaving", as used herein, refers to treating a substance with electromagnetic radiation with wavelengths ranging from about one meter to one millimetre and frequencies between 300 MHz and 300 GHz. In a particular embodiment, the method of the invention does not comprise a step of microwaving the substrate comprising cellulose and the enzyme, more particular for a period of time comprised between 10 and 180 minutes, for example, between 20 and 150 minutes, between 30 and 120 minutes, between 45 and 90 minutes, between 50 and 60 minutes.

The term "wet disk milling" or "WDM", as used herein, refers to a mechanical size reduction process that fibrillates the plant cell wall to nanoscale by the application of shear force and pressure under wet conditions. In a particular embodiment, the method of the invention does not comprise a step of treating the substrate comprising cellulose by wet disk milling, in particular, by wet disk milling with a disk clearance of 10 $\mu$m at a rotational speed of 1800 rpm.

The term "mechanical shearing", as used herein, refers to a process that directly or indirectly causes breakage of particles or limits the growth of particles.

The term "bleaching", as used herein, refers to treating a substance in order to lighting its colour or whitening it. The term bleaching includes oxidative bleaching, which not only removes coloured substances, but also residual lignin and other debris, and reductive bleaching, which does not cause the loss of fiber components. Oxidative bleaching is usually carried out using sodium hypochlorite, sodium chlorite or sulfuric acid. Reductive bleaching is usually done with sodium hydrosulphite. In a particular embodiment, the method of the invention does not comprise a step of bleaching the substrate comprising cellulose or the product obtained after contacting this substrate with the enzyme comprising the endoglucanase catalytic domain. In a more particular embodiment, the method of the invention does not comprise a step of bleaching the substrate comprising cellulose or the product obtained after contacting this substrate with the enzyme comprising the endoglucanase catalytic domain with NaOH and or NaClO$_2$.

In a particular embodiment, the method of the invention does not comprise any step directed to the chemical hydrolysis of the substrate comprising cellulose, and in particular, it does not comprise treating the substrate comprising cellulose with sulphuric acid and/or with hydrochloric acid.

In a particular embodiment, the enzyme comprising an endoglucanase catalytic domain is immobilized in a solid support. Non-limiting exemplary solid supports include polymers (such as agarose, sepharose, cellulose, nitrocellulose, alginate, Teflon, latex, acrylamide, nylon, plastic, polystyrene, silicone, polymethylmetacrylate (PMMA), etc.), glass, silica, ceramics, and metals. Such solid supports may take any form, such as particles (including microparticles), sheets, dipsticks, gels, filters, membranes, microfiber strips, tubes, wells, plates (such as microplates, including 6-well plates, 24-well plates, 96-well plates, 384-well plates, etc.), fibers, capillaries, combs, pipette tips, microarray chips, etc. In some embodiments, the surface of the solid support comprises an irregular surface, such as a porous, particulate, fibrous, webbed, or sintered surface. In some embodiments, a solid support is selected from a microplate, a microarray chip, and a microparticle. In some embodiments, a solid support is at least partially composed of a polymer.

*Crystalline nanocellulose*

The authors of the present invention have found that crystalline nanocellulose obtained by the method of the invention has a lower aspect ratio and reduced length that crystalline nanocellulose obtained by a method comprising using the same endoglucanase in the absence of a loosenin or a loosenin-like protein.

Therefore, in another aspect, the invention relates to crystalline nanocellulose obtained or obtainable by the method of the invention. Therefore, all the definitions, particular and preferred embodiments of the method of the invention fully apply to the crystalline nanocellulose of the invention.

In a particular embodiment, the crystalline nanocellulose of the invention has an average length comprised between 10 nm and 1000 nm, for example, between 20 nm and 800 nm, between 30 nm and 600 nm, between 50 nm and 500 nm, preferably between 150 nm and 350 nm, even more preferably, between 200 and 300 nm.

The length of the crystalline nanocellulose can be calculated using any suitable technique known by the skilled person, for example, by atomic force microscopy, as detailed in the examples herein included. Atomic force microscopy (ATM) is a technique that uses the interaction between the tip and the sample attractive-repulsion forces to create a deflection in the tip permitting to infer the imagines by mapping the deflections in each point of the sample.

As explained before, the crystalline nanocellulose of the invention preserve the structure of native cellulose, and therefore it is type I cellulose.

In a particular embodiment, the crystalline nanocellulose of the invention has a crystallite size comprised between 4.0 and 7.0 nm, for example between 4.2 and 6.5 nm, between 4.5 nm and 6 nm, between 4.8 and 5.5 nm, preferably between 4.2 nm and 6.5 nm, more preferably about 5.0 nm.

The crystallite size can be determined by any suitable method known by the skilled person, for example, by X-Ray diffraction as detailed in the examples herein included. The crystallite size can be measured from the diffractograms using the Scherrer's equation:

$$\beta = \kappa\lambda/\tau cos\theta \times 100$$

where $\lambda$ is the wavelength of the incident X-ray, $\theta$ is the angle of the (200) plane, $\beta$ is the full-width at half maximum of the (200) peak, $\tau$ is the crystallite size and $\kappa$ is a constant value.

In a particular embodiment, the crystalline nanocellulose of the invention has a maximum degradation temperature higher than 340°C, for example, at least 321°C, at least 342°C, at least 343°C, at least 344°C, at least 345°C or higher. The degradation temperature can be calculated by thermogravimetric analysis (TGA). The maximum degradation temperature (Td) is the minimum of the degradation peak in the derivative of thermogravimetric curves (DTG).

*Method for preparing a conductive ink*

In another aspect, the invention relates to a method for preparing a conductive ink comprising the following steps:

(i) mixing the crystalline nanocellulose of the invention with graphite and a first polar solvent, wherein the weight ratio of the crystalline nanocellulose to graphite is comprised from 1:100 to 1:20;
(ii) homogenising the mixture provided in step (i);
(iii) adding to the mixture obtained in step (ii) a second polar solvent having a boiling point boiling point equal to or higher than the first polar solvent provided in step (i); and
(iv) removing substantially all the first polar solvent provided in step (i) while leaving substantially all the second polar solvent added in step (iii).

The crystalline nanocellulose of the invention has been previously defined. All the particular and preferred embodiments of the crystalline nanocellulose of the invention also apply to the method for preparing a conductive ink.
The term "ink" includes any liquid capable of forming an image, a graphic, a pattern, etc., when applied to a print medium
In the context of the present invention, the term "conductive ink" refers to a material that allows the flow of charge (electrical current) in one or more directions. Electrical current is generated by the flow of negatively charged electrons, positively charged holes, and positive or negative ions in some cases. Therefore, the conductive ink is capable of conducting electricity, i.e., has electrical conductivity, or simply conductivity. Electrical conductivity (or conductivity, measured as Siemens per meter, S/m) is a fundamental property of a material that quantifies how strongly it conducts electric current.
Step (i) of the method for preparing a conductive ink comprises mixing the crystalline nanocellulose of the invention with graphite and a first polar solvent, wherein the weight ratio of the crystalline nanocellulose to graphite is comprised from 1:100 to 1:20.
In a preferred embodiment, the first polar solvent of the mixture of step (i) is selected from the group consisting of water, methanol, ethanol, propanol, isopropanol, butanol, tert-butanol, acetone, methyl ethyl ketone, 3-pentanone, 2-pentanone and mixtures thereof.
In another preferred embodiment, the first polar solvent of the mixture of step (i) is selected from the group consisting of water, methanol, ethanol, propanol, isopropanol, butanol, *tert*-butanol and mixtures thereof.
In another more preferred embodiment of the first aspect of the invention, the first polar solvent is a mixture of water and isopropanol; preferably in a 1:1 volume ratio.
The term "graphene" is to be interpreted as an allotrope of carbon in the form of a single layer of atoms in a two-dimensional hexagonal lattice, characterized by tightly packed carbon atoms and a $sp^2$ orbital hybridization. Graphene is a zero-gap semiconductor and displays remarkable electron mobility at room temperature, with reported values in excess of 15000 $cm^2 \times V^{-1} \times s^{-1}$. The term "graphite", as used herein, refers to many stacked layers of graphene, whereby the layers interact with each other through electronic interactions via the $\pi$ orbitals formed by the $sp^2$ carbon atoms in each layer.
In a preferred embodiment, the graphite is selected from the group consisting of synthetic graphite powder, natural graphite flakes, natural graphite powders and mixtures thereof; preferably it is synthetic graphite powder.
In another preferred embodiment of the first aspect of the invention, the graphite of the mixture of step (i) has a particle size of less than 30 $\mu$m.
The weight ratio of crystalline nanocellulose to graphite is comprised from 1:100 to 1:20. In certain particular embodiments, the weight ratio of crystalline nanocellulose to graphite is comprised from 1:98 to 1:30, preferably from 1.5:97 to 1:30, even more preferably, about 1.8:96.3.
Step (ii) of the method for producing the conductive ink of the invention comprises homogenising the mixture provided in step (i).
In a particular embodiment, step (ii) is carried out by sonicating with a mild bath or a tip or by shear mixing the mixture of step (i), more particularly, by sonicating with a mild bath.
Step (iii) of the method for producing the conductive ink of the invention comprises adding to the mixture obtained in step (ii) a second polar solvent having a boiling point boiling point equal to or higher than the first polar solvent provided in step (i).
In a particular embodiment, the second polar solvent of step (iii) is selected from the group consisting of water, 1,2-propanodiol, glycerol, diethylene glycol, 1,2-ethanodiol, terpineol and mixtures thereof.
In another particular embodiment, the second polar solvent of step (iii) is 1,2-propanodiol or water, preferably 1,2-propanodiol.
In a particular embodiment, the second polar solvent of step (iii) is 1,2-propanodiol in an amount such that the volume ratio 1,2-propanodiol to the first polar solvent provided in step (i) is from 1:5 to 1:10; preferably it is of 1:8. This advantageously allows producing inks with high viscosity.

In a particular embodiment, the second polar solvent of step (iii) is in an amount such that the volume ratio of said second polar solvent to the first polar solvent provided in step (i) is from 1:5 to 1:10.

In a particular embodiment, the second polar solvent of step (ii) and the first polar solvent of step (i) are the same solvent. In such embodiment, step (iv) is a concentration step in a manner that the volume of solvent remaining after the concentration step is from is from 0.1 to 0.2 the volume of solvent prior to step (v); preferably it is 0.125 times the volume of solvent prior to step (iv).

Step (iv) of the method for producing a conductive ink of the invention comprises removing substantially all the first polar solvent provided in step (i) while leaving substantially all the second polar solvent added in step (iii).

In a particular embodiment, step (iv) is carried out by heating and/or by applying vacuum, preferably by heating at a temperature of 100 °C.

In a particular embodiment, the method for producing the conductive ink further comprises a step previous to step (i), said previous step consisting of producing crystalline nanocellulose with the method of producing crystalline nanocellulose of the invention.

*Conductive ink*

The authors of the present invention have found that the graphene-based conductive ink obtained by the aforementioned method has some unexpected properties over other conductive inks, in particular over those obtained using crystalline nanocellulose obtained by enzymatic hydrolysis with the same endoglucanase in the absence of loosenin or loosenin-like protein. In particular, the conductive ink of the invention has decreased sheet resistance and resistivity and increased conductivity.

Therefore, in another aspect, the invention relates to a conductive ink obtainable or obtained or directly obtained by the method for producing a conductive ink of the invention. Therefore, all the definitions, particular and preferred embodiments of the method for producing a conductive ink fully apply to the conductive ink of the invention.

In a particular embodiment, the conductive ink as a sheet resistance (Rs) lower than 29.5 $\Omega/\square$, for example, lower than 29.0 $\Omega/\square$, lower than 25.0 $\Omega/\square$, lower than 20.0 $\Omega/\square$, lower than 15.0 $\Omega/\square$, lower than 10.0 $\Omega/\square$, lower than 8.0 $\Omega/\square$, preferably lower than 5.0 $\Omega/\square$, even more preferably about 3.0 $\Omega/\square$ or lower.

The sheet resistance can be calculated by using a four-point probe as explained in the examples herein included.

In a particular embodiment, the conductive ink of the invention has a resistivity lower than $6.3 \times 10^{-4} \Omega \times m$ for example, lower than $6.0 \times 10^{-4} \Omega \times m$, lower than $5.0 \times 10^{-4} \Omega \times m$, lower than $4.0 \times 10^{-4} \Omega \times m$, lower than $3.0 \times 10^{-4} \Omega \times m$, lower than $2.0 \times 10^{-4} \Omega \times m$, lower than $1.5 \times 10^{-4} \Omega \times m$, preferably about $1.0 \times 10^{-4} \Omega \times m$ or lower.

The resistivity can be calculated from the sheet resistance and thickness values using the formula included in the examples herein included.

In a particular embodiment, the conductive ink of the invention has a conductivity higher than $1.5 \times 10^3$ Siemens/m, for example, higher than $2.0 \times 10^3$ Siemens/m, higher than $3.0 \times 10^3$ Siemens/m, higher than $4.0 \times 10^3$ Siemens/m, higher $5.0 \times 10^3$ Siemens/m, higher than $6.0 \times 10^3$ Siemens/m, higher than $7.0 \times 10^3$ Siemens/m, higher than $8.0 \times 10^3$ Siemens/m preferably about $9.0 \times 10^3$ Siemens/m or higher.

The invention will be described by way of the following examples which are to be considered as merely illustrative and not limitative of the scope of the invention.

## EXAMPLES

## MATERIALS AND METHODS

### Materials

Commercial cellulose nanocrystals (CNC) produced by sulfuric acid treatment were purchased from the University of Maine, USA (Lot# 2014- FPL-CNC-065).

### Protein expression and purification

The ancestral endoglucanase, LFCA_EG described in Barruetabeña et al. (supra), was synthesized and codon optimized for *Escherichia coli* cell expression. LFCA_EG was cloned in pQE80L expression vector and transformed in *E. coli* BL21 (DE3) (Life Technologies) for protein expression. Cells were incubated in 500 mL of LB medium at 37 °C until OD600 reached 0.6 and 1 mM of IPTG was added to the medium for protein induction overnight at 18 °C. Cells were pelleted by centrifugation at 5000 g. Pellets were suspended in extraction buffer (Tris 50 mM, NaCl 200 mM, pH 8, imidazole 10 mM) supplemented with EDTA-free protease inhibitor (Thermo Fisher Scientific) and then sonicated for 3 cycles over 10 min at 30% amplitude. Cell debris was separated by ultracentrifugation at 33,000 G for 1 h at 4°C. For purification, the supernatants were mixed with 1 mL (50/50, v/v) Ni-NTA agarose beads (Thermo Fisher Scientific) and incubated for 1 h in a roller shaker. The bound protein was then washed with wash buffer (NaCl 200 mM, Tris 50 mM, 20mM imidazole, pH 8) and eluted in six column volumes of elution buffer (NaCl 200 mM, Tris 50 mM, 300 mM imidazole, pH 8). The purified protein was exchanged to 50 M HEPES, 150 mM NaCl, pH 7.5 and concentrated to 10-15 mg/mL using Vivaspin® 20 Ultrafiltration Units (30 kDa) from Sartorius. The protein purity was verified by SDS-PAGE before flash freezing and storing the protein at - 80°C. Protein concentration was determined by measuring absorption at 280 nm using a NanoDrop™ Lite Spectrophotometer (Thermo Fischer Scientific).

*Pca*LOOL12 from *Phanerochaete carnosa* (GenBank code EKM51974.1) was recombinantly produced in *Pichia pastoris* strain SMD1168H according to the manufacturer's instructions (Invitrogen, Thermo Fisher Scientific) and as previously described (Dahiya, D. et al., Bioresource Technology 2024, 394, 130188). Briefly, transformants were grown for up to three days on YPD-agar containing 100 $\mu$g/mL zeocin before being transferred to 300 mL of buffered glycerol complex medium (BMGY) in 2 L non-baffled Erlenmeyer flasks; liquid cultures in BMGY were grown at 30 °C and 180 rpm until the OD600nm was ~ 25. The cells obtained from this preculture were used to inoculate a 7 L Sterilizable-In-Place (SIP) Fermenter (BIOSTAT® Cplus from Sartorius) containing minimal basal salts media (BSM) and trace salts prepared according to the manufacturer's guidelines for Pichia Fermentation (Invitrogen™, Thermo Fisher Scientific). The bioreactor cultivation was performed at 30 °C and the pH was maintained at pH 6.0 using 14% ammonium hydroxide throughout the production. The bioreactor parameters were set as follows: air flow rate in between 40 and 60% with volume of air per volume of liquid per minute (VVM) between 0.5 and 1.0, oxygen at 35% and stirring at 900 rpm. The oxygen was controlled using an automatic cascade with stirring and gas flow functioning on a Proportional-Integral-Derivative (PID) controller. Following the batch phase, 4% (w/v of media) glycerol was added to further increase cell density before inducing PcaLOOL12 production through methanol fed batch phase (MFBP) performed at 20 °C using a methanol flow rate of 22.75 mL/h for 110 h. The pH of the medium in the bioreactor was then adjusted to 7.8 using 4M sodium hydroxide before removing the cells by centrifuged. The recovered supernatant was filtered through a 0.45 $\mu$m polyethersulfone (PES) membrane from Millipore before purifying the recombinant 6xHis-tagged PcaLOOL12 protein using Ni-NTA affinity resin (Qiagen; Cat.# 30230). The purified protein was exchanged to 20 mM sodium acetate buffer (pH 6.0) and concentrated to 10-15 mg/mL using Vivaspin® 20 Ultrafiltration Units (5 kDa) from Sartorius. The protein purity was verified by SDS-PAGE before flash freezing and finally storing the protein at - 80 °C. Protein concentration was determined by measuring absorption at 280 nm using a NanoDrop™ Lite Spectrophotometer (Thermo Fischer Scientific).

## Enzymatic preparation of nanocellulose

Enzyme treatments were performed in milliQ water (40 mL, pH of ~ 6) and comprised 0.4 g Whatman® filter paper Grade 1 from Merck (Sigma-Aldrich) prepared as 1 cm$^2$ squares. Reactions were amended with 5 mg/g substrate of LFCA_EG alone, 5 mg/g substrate of LFCA_EG plus the equivalent mole amount of *Pca*LOOL12 (1.88 mg/g substrate), and 1.88 mg/g substrate PcaLOOL12 alone, and then incubated at 40 °C with agitation (180 rpm) for 24 h before being stopped by transferring the reaction to ice for 30 min. Samples were then sonicated with a micro-tip sonicator UPH100H ultrasonic processor (Hielscher) for 25 min at 30-40% of intensity, and the resulting nanocellulose was washed by sequential sedimentation and centrifugation. Specifically, following sonication the samples were incubated at RT for 45 min to sediment fiber and microfibers. The supernatant was recovered and processed by ultracentrifugation at 33,000$\times$g for 1 h; the nanocellulose pellet was isolated and concentrated to 20-30 mg/mL in milliQ water.

## Characterization of nanocelluloses

*Atomic force microscopy of nanocellulose preparations.* The dimensions of enzymatically produced nanocelluloses were compared to commercial CNC using atomic force microscopy (Multimode 8 AFM from Bruker). Samples for AFM were prepared by sonicating each nanocellulose preparation for 2 min (2 sec. on-off) at 40% amplitude prior to centrifugation at 4000 rpm for 30 s. Approximately 300-500 $\mu$L of supernatant was transferred to a 2 mL Eppendorf tube and used to prepare 1:2, 1:5, and 1:10 dilutions of the nanocellulose in milliQ water. Each dilution was briefly centrifuged (30 s at 3000 rpm) before ~ 100 mL of nanocellulose solution was used to spin-coat a PEI-treated silica wafer (Okmetic Oy, Finland) for 40 s at 300 rpm. Critically, before spin-coating, each silica wafer was washed with 0.1M NaOH, rinsed with deionized water and then ozone treated for 15-20 min before coating with 1% cationic polyethyleneimine (PEI) [38,39]. Following spin-coating, the cellulose-coated wafers were dried overnight at room temperature in small petri dishes. AFM images were then obtained in tapping mode in air using aluminum-coated n-type silicon cantilevers (BRUKER NCHV-A) with a typical probe radius of 8 nm, force constant of 40 N m-1, and nominal resonance frequency of 320 kHz. Approximately 100 nanocellulose particles were measured from different images using Mountain's lab software. No image processing was performed except the use of flattening feature.

*Zeta potential measurement.* The zeta potential ($\zeta$, mV) of the obtained samples (0.1 wt%) were measured using a Malvern Zetasizer Nano ZS90. The voltage of the instrument was set at 40V with a laser wavelength of 633 nm. All the samples were sonicated for 2 min with 2 sec. on-off at 40% amplitude and measurements were obtained in triplicates.

*XOR, FTIR and TGA analysis.* FTIR spectra were collected using a Nicolet Nexus spectrometer equipped with a MKII Golden Gate accessory (Specac) with a diamond crystal at a nominal incidence angle of 45° and ZnSe lens. Spectra were measured in attenuated reflection (ATR) mode between 4000 and 650 cm$^{-1}$, with averaging 32 scans and a resolution of 4 cm$^{-1}$. X-ray diffraction (XRD) was used for the analysis of the cellulose crystalline structure, crystallinity, and crystallite size. X-ray powder diffraction patterns were collected by using a Philips X'pert PRO automatic diffractometer operating at 40 kV and 40 mA, in theta-theta configuration, a secondary monochromator with Cu-K$\alpha$ radiation ($\lambda$ = 1.5418 Å) and a PIXcel solid state detector (active length 2$\theta$ =3.347°). Data were collected from 5° to 50° 2$\theta$ (step size 0.026 and time per step 80 s) at room temperature. A fixed divergence and antiscattering slit, which gives a constant volume of sample illumination, were used. The crystallite size was measured from the diffractograms using the Scherrer's Eq. (Eq.1):

$$\beta = \frac{k\lambda}{\tau \cos\theta} \, x \, 100 \qquad\qquad (1)$$

where A is the wavelength of the incident X-ray, $\theta$ is the angle of the (200) plane, $\beta$ is the full width at half maximum of the (200) peak, $\tau$ is the crystallite size and k is a constant value.

Thermogravimetric analysis (TGA) was performed using TGA/SDTA 851 Mettler Toledo equipment. 5 mg of the samples were used and heated from 30 to 800 °C in a nitrogen atmosphere at a scanning rate of 10 °C/min. The maximum degradation temperature (Td) is the minimum of the degradation peak in the derivative of thermogravimetric curves (DTG).

*Measurement of nanocellulose yield.* Nanocellulose conversion was calculated by weighing the freeze-dried nanocellulose from each hydrolysis reaction in triplicate and using the following equation to calculate the yield conversion rate (Eq. 2):

$$\text{Nanocellulose yield (\%) = (Nanocellulose mass / Initial cellulose mass) x 100} \qquad (2)$$

**Preparation and printing of inks.**

For the preparation of conductive inks, the nanocellulose preparations were mixed with graphite with a mass ratio of 96.3:1.8 graphite: CNC. The mixture was dissolved in water and isopropanol 1:1 (v/v) and sonicated for 4 h at 40 % amplitude. After that, 1,2-propanodiol was added to the mixture resulting in a 1:8 proportion relative to the mixture. Finally, both the water and the isopropanol were evaporated at 100 °C under agitation, resulting in a viscous grey fluid that constituted the conductive ink.

To analyze the electrical properties of the inks, samples were evenly spread onto a circular mold placed on a cellulose-based surface. The ink was applied using a rolling pin, with the process repeated twice to create a film consisting of two layers of conductive ink. After each layer was applied, the films were dried at 100 °C for 15 min and 30 min after the first and second layers, respectively. The two-layered films were then mechanically compacted to secure the ink onto the cellulose surface.

**Electrochemical characterization.**

Three independent films prepared with each ink was analyzed using a four-point probe at 10 mA (JANDEL Model RM3-AR). The thickness of each film was determined using a micrometre device at five different points of each film. The thickness was blacked with the average of the paper background in five positions around the film.

All other electrical properties were estimated with the experimental data of resistance and thickness.

Resistivity ($\rho$) ($\Omega \times$ m) (Eq. 3):

$$\rho = \text{Resitance} \, \frac{Transversal\ area}{distance} \qquad\qquad (3)$$

$$Transversal\ area = \frac{distance\ between\ multimeter\ tips}{thickness} = \frac{0.01\ m}{thickness\ (m)}$$

Conductivity ($\sigma$) (Siemens/m) (Eq. 4):

$$\sigma = \frac{1}{\rho} \qquad\qquad (4)$$

Sheet-resistance ($R_s$) ($\Omega/\square$) (Eq. 5):

$$R_s = \frac{\rho}{thickness}$$

## RESULTS

Enzymatic nanocellulose production and characterization

The catalytic domain of an ancestral endoglucanase (LFCA_EG) combined with a present-day family 30 carbohydrate-binding module (CBM30) from C. *thermocellum* (LFCA_EG + CBM) was previously shown to produce cellulose nanocrystals at higher yield and better aspect ratio than those produced by the LFCA_EG without CBM. Despite the improved performance of LFCA_EG over the parent EG, the resulting CNC was still approximately two times longer than CNCs prepared using acid hydrolysis. It is conceivable that the performance of an ancestral endoglucanase in preparing CNCs could be enhanced through amending corresponding reactions with proteins expected to disrupt cellulose fiber networks, thereby increasing the enzyme access to the cellulose substrate. To test this hypothesis, the potential of loosenin *Pca*LOOL12 to enhance the ability of the ancestral endoglucanase LFCA_EG to obtain nanocrystals of reduced size was assessed. In these experiments, LFCA_EG without the appended CBM30 was used to evaluate the impact of PcaLOOL12 to prevent possible CBM-induced structural disruption of the cellulose substrate.

After 24h at 40 °C, the reactions comprising LFCA_EG + *Pca*LOOL12 had produced smaller enzymatically prepared CNC particles (i.e., EnCNC) compared to LFCA_EG alone (Fig. 1). Specifically, a homogenous EnCNC suspension with an average length of 250 ± 100 nm was produced by the LFCA_EG + *Pca*LOOL12 treatment in comparison with the average length of 650 ± 390 nm obtained from LFCA_EG alone. The size of EnCNC particles produced using LFCA_EG + *Pca*LOOL12 was thus within the range of the commercial AcCNC produced through acid hydrolysis (Fig. 1). Moreover, the nanocellulose yield was approximately 1.5 times higher in reactions comprising LFCA_EG + *Pca*LOOL12 compared to LFCA_EG alone.

Besides size and yield, the presence of *Pca*LOOL12 impacted the morphology of EnCNC. Whereas EnCNC produced by LFCA_EG exhibited a needle-like morphology, LFCA_EG + *Pca*LOOL12 EnCNC crystals, due to their reduced size, adopted a ribbon-like morphology similar to that exhibited by AcCNC (Fig. 1). The stability of water-dispersed CNC preparations were compared by measuring corresponding zeta potential values ($\zeta$). The zeta potential values for LFCA_EG, LFCA_EG + *Pca*LOOL12 and AcCNC were -33, -38 and -48 mV, respectively, confirming that the addition of *Pca*LOOL12 did not reduce the dispersion stability of EnCNC and instead slightly improved this property (Table 2).

**Table 2.** Average size of the nanocellulose produced from filter paper using different protein combinations, and the nanocellulose produced by acid sulfuric treatment. The average size and S.D. were calculated from at least 100 particles. For zeta-potential measurements and yield measurements, n = 3.

| Treatment | Mean size (nm) | Std. dev. |
|---|---|---|
| **Ac_CNC** | 143.0 | 70 |
| **LFCA_EG** | 644.2 | 390 |
| **LFCA_EG + *Pca*LOOL12** | 248.9 | 110 |

| | Zeta potential (mV) | Std. dev. |
|---|---|---|
| **Ac_CNC** | -47.8 | 0.7 |
| **LFCA EG** | -33.1 | 0.8 |
| **LFCA_EG + *Pca*LOOL12** | -38.4 | 1.0 |

| | Yield conversion (%) | Std.dev. |
|---|---|---|
| **LFCA EG** | 22.3 | 1.5 |

(continued)

| | Yield conversion (%) | Std.dev. |
|---|---|---|
| LFCA_EG + *Pca*LOOL12 | 34.2 | 2.3 |

The physicochemical properties of LFCA_EG and LFCA_EG + *Pca*LOOL12 CNCs were further evaluated using FTIR and X-ray diffraction (XRD). (Fig. 2a). XRD analysis revealed diffractograms of cellulose I in both samples, having a similar crystallite size (5 nm, Table 3) that was slightly higher than that displayed by AcCNC (4 nm) (Alonso-Lerma, B. et al., Communications Materials 2020, 1 (1), 57). This difference correlates with the transformation from cellulose I to cellulose II during acidic treatment [42]. Furthermore, as anticipated, the FTIR spectra were nearly identical, with no signals at 3486 and 3441 cm$^{-1}$ (marked with dotted lines) that characterize the cellulose II structure (Fig. 2b). The thermogravimetric analysis (TGA, Figure 3c, d) indicated that CNCs prepared using LFCA_EG + *Pca*LOOL12 (344.22 °C, Table 3) had the highest degradation temperature, followed by CNCs prepared using LFCA_EG (340 °C, Table 4). These values contrast with the thermal stability of AcCNC (298 °C) (Alonso-Lerma, B. et al., *supra*), demonstrating the superior thermostability of the EnCNC samples.

Table 3. Crystallite size (size of a single crystal within a polycrystalline material) of the CNC produced by LFCA_EG and LFCA_EG + *Pca*LOOL12 after 24 h. The crystallite size was calculated by Scherrer's equation. n= 1.

| Sample | Crystallite size [nm] |
|---|---|
| LFCA EG | ~5 |
| LFCA_EG + *Pca*LOOL12 | ~5 |

Table 4. TGA analysis. Maximum thermal degradation temperature (Td) and char residue (%) of crystals produced LFCA_EG and LFCA_EG + *Pca*LOOL12 after 24 h. n=1.

| Treatment | Td (°C) | Char residue (wt%) |
|---|---|---|
| LFCA EG | 340.5 | 12.92% |
| LFCA EG + *Pca*LOOL12 | 344.22 | 16.45% |

Overall, the combination of LFCA_EG with *Pca*LOOL12 improved the production of crystals with a size, morphology and stability similar to those exhibited by AcCNC. Accordingly, the performance of this new EnCNC preparation was evaluated in applications for conductive inks.

**Application of EnCNC to produce conductive inks**

Graphene is a lightweight material with excellent mechanical and conductivity properties, rendering it an attractive component for the formulation of non-toxic conductive inks used in trackable packaging materials. However, producing processable graphene flakes in large quantities is an ongoing challenge. Recently, EnCNC has been identified as an effective exfoliation agent to graphite for the production of graphene flakes. In addition to the exfoliation by mechanical shear strength during the sonication of a mixture with graphite, CNC helps the colloidal stabilization of the carbon. Therefore, by sonication of graphite with EnCNC it is possible obtain an aqueous solution containing graphene. This process is used to create graphene-based conductive ink through a cost-effective, eco-friendly and scalable process.

It is hypothesized that the decreased size of the new EnCNC will enhance well-structured graphene formation, thus creating inks with superior conductivity properties. To test this hypothesis, prototype conductive inks were made using the reduced-size EnCNC prepared using LFCA_EG + PcaLOOL12. Circular tags were then printed using screen printing with these inks (Fig. 3a). The sheet resistance ($R_s$) is expressed in ohms per square ($\Omega/\square$) and was measured using a four-point probe. It was observed that the $R_s$ of the ink produced with reduced-size crystals in the presence of PcaLOOL12 was $3.19 \pm 0.89$ $\Omega/\square$, nearly ten times lower than that of the LFCA_EG ink ($29.64 \pm 2.70$ $\Omega/\square$) (Fig. 3b; Table 4). Since $R_s$ is inversely proportional to the thickness (see Eq. 5 in materials and methods), the resistivity of the material was calculated to eliminate the influence of the printed film's width (Fig. 3c; Table 5). Considering this, the resistivity of LFCA_EG + *Pca*LOOL12 ink was $(1.15 \pm 0.32) \times 10^{-4}$ $\Omega \times$ m, nearly 5 times lower than that of the LFCA_EG ink ($6.32 \pm 0.57$) $\times 10^{-4}$ $\Omega \times$ m, and the conductivities of LFCA_EG + *Pca*LOOL12 ink was $(9.26 \pm 3.00) \times 10^3$ Siemens/m, 5.8 times higher than that of the LFCA_EG ink ($1.59 \pm 0.14$) $\times 10^3$ Siemens/m (Fig. 3d; Table 6). These results demonstrated that inks with enhanced properties can be obtained using reduced-size nanocrystals.

| LFCA EG | | | | | |
|---|---|---|---|---|---|
| | Ω/□ Fw | Ω/□ Rev | Ω/□ Average | $\rho$ (Ω × m × $10^{-4}$) | $\sigma$ (Siemens/m × $10^3$) |
| Tag 1 | 27.32 | 27.38 | 27.35 | 5.83 | 1.71 |
| Tag 2 | 28.98 | 28.95 | 28.97 | 6.18 | 1.62 |
| Tag 3 | 31.61 | 32.36 | 32.62 | 6.96 | 1.44 |
| Average | | | 29.64 ± 2.70 | 6.32 ± 0.57 | 1.59 ± 0.14 |

Table 5. Electrical properties of conductive bio-inks prepared using EnCNC in the presence of LFCA_EG or LFCA_EG + PcaLOOL12 (mean ± S.D., n = 3 independent printed tags).

| LFCA_EG + *Pca*LOOL12 | | | | | |
|---|---|---|---|---|---|
| Tag 1 | 3.47 | 3.48 | 3.48 | 1.25 | 7.98 |
| Tag 2 | 3.90 | 3.90 | 3.90 | 1.41 | 7.11 |
| Tag 3 | 2.18 | 2.19 | 2.18 | 0.79 | 12.70 |
| Average | | | 3.19 ± 0.89 | 1.15 ± 0.32 | 9.26 ± 3.00 |

Table 6. Thickness of conductive inks using EnCNC prepared using LFCA_EG or LFCA_EG + *Pca*LOOL12 (mean ± S.D., n = 3 independent printed tags with measurements at 5 points in each).

| LFCA EG | | | |
|---|---|---|---|
| | Tag 1 (μm) | Tag 2 (μm) | Tag 3 (μm) |
| Position 1 | 18 | 24 | 15 |
| Position 2 | 17 | 20 | 20 |
| Position 3 | 24 | 26 | 25 |
| Position 4 | 28 | 22 | 21 |
| Position 5 | 24 | 19 | 17 |
| Average per Tag | 22.2 | 22.2 | 19.6 |
| Overall average | | | 21.3 ± 1.5 |

| LFCA_EG + *Pca*LOOL12 | | | |
|---|---|---|---|
| | Tag 1 (μm) | Tag 2 (μm) | Tag 3 (μm) |
| Position 1 | 42 | 36 | 41 |
| Position 2 | 39 | 37 | 37 |
| Position 3 | 27 | 40 | 36 |
| Position 4 | 34 | 37 | 34 |
| Position 5 | 32 | 34 | 35 |
| Average per Tag | 34.8 | 36.8 | 36.6 |
| Overall average | | | 36.1 ± 1.1 |

## Claims

1. A combination comprising:

(v) an enzyme comprising an endoglucanase catalytic domain, wherein the endoglucanase catalytic domain comprises a sequence selected from the group consisting of SEQ ID NO: 1, SEQ ID NO: 2, SEQ ID NO: 3, SEQ ID NO: 4 and a functionally equivalent variant thereof that substantially maintains its endoglucanase activity and
(vi) a loosenin or a loosenin-like protein.

2. The combination of claim 1, wherein component (ii) is a loosenin-like protein from *Phanerochaete carnosa.*

3. The combination of claim 2, wherein the loosenin-like protein is the loosenin-like protein from *Phanerochaete carnosa* having the sequence of SEQ ID NO: 5 or a functionally equivalent variant thereof.

4. The combination according to claim 1, wherein the functionally equivalent variant of the endoglucanase catalytic domain has at least 70% sequence identity with the sequence of SEQ ID NO: 1, 2, 3 or 4.

5. The combination according to any one of claims 1 to 4, wherein components (i) and (ii) are included in the same or separate formulations.

6. The combination according to any one of claims 1 to 5, wherein the combination comprises:

(i) an enzyme comprising an endoglucanase catalytic domain, wherein the endoglucanase catalytic domain comprises the sequence of SEQ ID NO: 1, and
(ii) a loosenin-like protein, wherein the loosenin-like protein comprises the sequence of SEQ ID NO: 5.

7. A method for producing crystalline nanocellulose comprising contacting a substrate comprising cellulose with the combination according to any one of claims 1 to 6 under suitable conditions for hydrolyzing cellulose.

8. The method according to claim 7, wherein the suitable conditions for hydrolyzing cellulose comprise contacting the substrate and the combination at a temperature of approximately 40°C and a pH between approximately 4.5 to 6.5 during at least approximately 24 hours.

9. Crystalline nanocellulose obtainable by the method according to claims 7 or 8.

10. A method for preparing a conductive ink comprising the following steps:

(i) mixing the crystalline nanocellulose according to claim 9 with graphite and a first polar solvent, wherein the weight ratio of the crystalline nanocellulose to graphite is comprised from 1:100 to 1:20;
(ii) homogenising the mixture provided in step (i);
(iii) adding to the mixture obtained in step (ii) a second polar solvent having a boiling point boiling point equal to or higher than the first polar solvent provided in step (i); and
(iv) removing substantially all the first polar solvent provided in step (i) while leaving substantially all the second polar solvent added in step (iii).

11. The method according to claim 10, wherein the weight ratio of the crystalline nanocellulose to graphite is approximately 1.8:96.3.

12. The method according to claims 10 or 11, wherein the first polar solvent of the mixture of step (i) is a mixture of water and isopropanol in a 1:1 volume ratio.

13. The method according to any one of claims 10 to 12, wherein step (ii) is carried out by sonication.

14. The method according to claim 15, wherein the second polar solvent is 1,2-propanodiol, and wherein 1,2-propanodiol is added in an amount such that the volume ratio 1,2-propanodiol to the first polar solvent provided in step (i) is from 1:5 to 1:10; preferably it is of 1:8.

15. A conductive ink obtainable by the method according to any one of claims 10 to 14.

FIG. 1

FIG. 2

FIG. 3

**b**

**c**

**d**

FIG. 3 (CONT.)

Europäisches
Patentamt

European
Patent Office

Office européen
des brevets

# EUROPEAN SEARCH REPORT

**Application Number**

EP 24 38 3305

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | EP 3 872 173 A1 (CIC NANOGUNE ASOCIACION CENTRO DE INVESTIG COOPERATIVA EN NANOCIENCIAS) 1 September 2021 (2021-09-01) | 9-15 | INV.<br>C12N9/42<br>C07K14/375<br>C12P19/04 |
| Y | * the whole document *<br>* examples 1-8 *<br>* claims 1-15 * | 1-15 | C12P19/14<br>C08B15/08<br>C09D11/08<br>C09D11/102 |
| X | EP 3 872 172 A1 (CIC NANOGUNE ASOCIACION CENTRO DE INVESTIG COOPERATIVA EN NANOCIENCIAS) 1 September 2021 (2021-09-01) | 9-15 | C09D11/14<br>C09D11/52 |
| Y | * the whole document *<br>* examples 1-4 *<br>* claims 1-15 * | 1-15 | |
| Y | EP 3 502 126 A1 (CIC NANOGUNE ASOCIACION CENTRO DE INVESTIG COOPERATIVA EN NANOCIENCIAS) 26 June 2019 (2019-06-26)<br>* the whole document *<br>* claims 1-5 * | 1-15 | |
| | | | **TECHNICAL FIELDS SEARCHED (IPC)** |
| Y,D | ALONSO-LERMA BORJA ET AL: "High performance crystalline nanocellulose using an ancestral endoglucanase", COMMUNICATIONS MATERIALS, vol. 1, no. 1, 12 August 2020 (2020-08-12), pages 1-10, XP093272897, ISSN: 2662-4443, DOI: 10.1038/s43246-020-00055-5 Retrieved from the Internet: URL:https://www.nature.com/articles/s43246-020-00055-5><br>* the whole document *<br>* abstract * | 1-15 | C12N<br>C07K<br>C12P<br>C08B<br>C09D<br>C09J |

-/--

The present search report has been drawn up for all claims

| Place of search | Date of completion of the search | Examiner |
|---|---|---|
| The Hague | 2 May 2025 | van de Kamp, Mart |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
................................................................
& : member of the same patent family, corresponding document

EPO FORM 1503 03.82 (P04C01)

page 1 of 6

Europäisches
Patentamt

European
Patent Office

Office européen
des brevets

## EUROPEAN SEARCH REPORT

**Application Number**

EP 24 38 3305

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | Lerma Borja Alonso: "Isolation, characterization and applications of nanocellulose produced by ancestral enzymes", Thesis (partial) , 2019, pages 1-36, 205-251, XP055919605, Retrieved from the Internet: URL:https://addi.ehu.es/bitstream/handle/1 0810/42554/TESIS_ALONSO_LERMA_BORJA.pdf?se quence=2 [retrieved on 2022-05-10] * the whole document * | 1-15 | |
| Y,D | BARRUETABEÑA NEREA ET AL: "Resurrection of efficient Precambrian endoglucanases for lignocellulosic biomass hydrolysis", COMMUNICATIONS CHEMISTRY, vol. 2, no. 1, 1 July 2019 (2019-07-01), pages 1-13, XP093272895, ISSN: 2399-3669, DOI: 10.1038/s42004-019-0176-6 Retrieved from the Internet: URL:https://www.nature.com/articles/s42004 -019-0176-6> * the whole document * * abstract * | 1-15 | |
| Y | WO 2017/121902 A1 (CIC NANOGUNE – ASOCIACIÓN CENTRO DE INVESTIGACIÓN COOP EN NANOCIENCIAS) 20 July 2017 (2017-07-20) * the whole document * * claims 1-17 * | 1-15 | TECHNICAL FIELDS SEARCHED (IPC) |

-/--

The present search report has been drawn up for all claims

| Place of search | Date of completion of the search | Examiner |
|---|---|---|
| The Hague | 2 May 2025 | van de Kamp, Mart |

EPO FORM 1503 03.82 (P04C01)

**EUROPEAN SEARCH REPORT**

**Application Number**

EP 24 38 3305

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | MONSCHEIN MAREIKE ET AL: "Loosenin-Like Proteins from Phanerochaete carnosa Impact Both Cellulose and Chitin Fiber Networks", APPLIED AND ENVIRONMENTAL MICROBIOLOGY, vol. 89, no. 1, 16 January 2023 (2023-01-16), pages 1-15, XP093273115, ISSN: 0099-2240, DOI: 10.1128/aem.01863-22 Retrieved from the Internet: URL:https://pmc.ncbi.nlm.nih.gov/articles/PMC9888185/pdf/aem.01863-22.pdf> * the whole document * * abstract * | 1-15 | |
| Y | MONSCHEIN MAREIKE ET AL: "PACER: a novel 3D plant cell wall model for the analysis of non-catalytic and enzymatic responses", BIOTECHNOLOGY FOR BIOFUELS AND BIOPRODUCTS, vol. 15, 30, 16 March 2022 (2022-03-16), pages 1-11, XP093273452, ISSN: 2731-3654, DOI: 10.1186/s13068-022-02128-8 Retrieved from the Internet: URL:https://link.springer.com/article/10.1186/s13068-022-02128-8/fulltext.html> * the whole document * * abstract * | 1-15 | TECHNICAL FIELDS SEARCHED (IPC) |

-----

-----

-/--

The present search report has been drawn up for all claims

| Place of search | Date of completion of the search | Examiner |
|---|---|---|
| The Hague | 2 May 2025 | van de Kamp, Mart |

## DOCUMENTS CONSIDERED TO BE RELEVANT

| Category | Citation of document with indication, where appropriate, of relevant passages | Relevant to claim | CLASSIFICATION OF THE APPLICATION (IPC) |
| --- | --- | --- | --- |
| Y | SUZUKI HITOSHI ET AL: "Sequence diversity and gene expression analyses of expansin-related proteins in the white-rot basidiomycete,Phanerochaete carnosa", FUNGAL GENETICS AND BIOLOGY, vol. 72, 28 May 2014 (2014-05-28), pages 115-123, XP029084992, ISSN: 1087-1845, DOI: 10.1016/J.FGB.2014.05.008 * the whole document * * abstract * | 1-15 | |
| Y | Anonymous: "hypothetical protein PHACADRAFT_262410 [Phanerochaete carnosa HHB-1011 - Protein - NCBI", , 18 March 2015 (2015-03-18), XP093273122, Retrieved from the Internet: URL:https://www.ncbi.nlm.nih.gov/protein/ekm51974 * abstract * | 1-15 | |
| Y | CEBREIROS FLORENCIA ET AL: "Enhancing cellulose nanofibrillation of eucalyptus Kraft pulp by combining enzymatic and mechanical pretreatments", CELLULOSE, vol. 28, no. 1, 27 October 2020 (2020-10-27), pages 189-206, XP037341589, ISSN: 0969-0239, DOI: 10.1007/S10570-020-03531-W * the whole document * * abstract * | 1-15 | TECHNICAL FIELDS SEARCHED (IPC) |

-/--

The present search report has been drawn up for all claims

| Place of search | Date of completion of the search | Examiner |
| --- | --- | --- |
| The Hague | 2 May 2025 | van de Kamp, Mart |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

Application Number

EP 24 38 3305

Europäisches
Patentamt

European
Patent Office

Office européen
des brevets

# EUROPEAN SEARCH REPORT

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y,D | HU ET AL: "Enzyme mediated nanofibrillation of cellulose by the synergistic actions of an endoglucanase, lytic polysaccharide monooxygenase (LPMO) and xylanase", SCIENTIFIC REPORTS, vol. 8, 16 February 2018 (2018-02-16), pages 1-8, XP002794887, DOI: 10.1038/S41598-018-21016-6 * the whole document * * abstract * | 1-15 | |
| Y | SQUINCA PAULA ET AL: "The use of enzymes to isolate cellulose nanomaterials: A systematic map review", CARBOHYDRATE POLYMER TECHNOLOGIES AND APPLICATIONS, vol. 3, 100212, 27 April 2022 (2022-04-27), pages 1-15, XP093273493, ISSN: 2666-8939, DOI: 10.1016/j.carpta.2022.100212 * the whole document * * abstract * | 1-15 | |
| X | SANCHEZ-DUENAS LEIRE ET AL: "A Review on Sustainable Inks for Printed Electronics: Materials for Conductive, Dielectric and Piezoelectric Sustainable Inks", MATERIALS, vol. 16, 3940, 24 May 2023 (2023-05-24), pages 1-22, XP093273669, ISSN: 1996-1944, DOI: 10.3390/ma16113940 * the whole document * | 10-15 | TECHNICAL FIELDS SEARCHED (IPC) |

-/--

The present search report has been drawn up for all claims

| Place of search | Date of completion of the search | Examiner |
|---|---|---|
| The Hague | 2 May 2025 | van de Kamp, Mart |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

................................................................

& : member of the same patent family, corresponding document

EPO FORM 1503 03.82 (P04C01)

Europäisches
Patentamt

European
Patent Office

Office européen
des brevets

## EUROPEAN SEARCH REPORT

**Application Number**

EP 24 38 3305

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| T | JABALERA YLENIA ET AL: "Impact of loosenins on the enzymatic preparation of cellulose nanocrystals", CARBOHYDRATE POLYMERS, vol. 357, 123469, 3 March 2025 (2025-03-03), pages 1-9, XP087736957, ISSN: 0144-8617, DOI: 10.1016/J.CARBPOL.2025.123469 [retrieved on 2025-03-03] * the whole document * * abstract * ----- | 1-15 | |
| | | | **TECHNICAL FIELDS SEARCHED (IPC)** |

The present search report has been drawn up for all claims

| Place of search | Date of completion of the search | Examiner |
|---|---|---|
| The Hague | 2 May 2025 | van de Kamp, Mart |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

page 6 of 6

## ANNEX TO THE EUROPEAN SEARCH REPORT
## ON EUROPEAN PATENT APPLICATION NO.

EP 24 38 3305

This annex lists the patent family members relating to the patent documents cited in the above-mentioned European search report.
The members are as contained in the European Patent Office EDP file on
The European Patent Office is in no way liable for these particulars which are merely given for the purpose of information.

02-05-2025

| Patent document cited in search report | | Publication date | Patent family member(s) | | Publication date |
|---|---|---|---|---|---|
| EP 3872173 | A1 | 01-09-2021 | EP | 3872173 A1 | 01-09-2021 |
| | | | EP | 4110911 A1 | 04-01-2023 |
| | | | WO | 2021170789 A1 | 02-09-2021 |
| EP 3872172 | A1 | 01-09-2021 | EP | 3872172 A1 | 01-09-2021 |
| | | | EP | 4110910 A1 | 04-01-2023 |
| | | | WO | 2021170770 A1 | 02-09-2021 |
| EP 3502126 | A1 | 26-06-2019 | BR | 112020012180 A2 | 24-11-2020 |
| | | | EP | 3502126 A1 | 26-06-2019 |
| | | | EP | 3728297 A1 | 28-10-2020 |
| | | | US | 2020392542 A1 | 17-12-2020 |
| | | | WO | 2019121719 A1 | 27-06-2019 |
| WO 2017121902 | A1 | 20-07-2017 | CA | 3011534 A1 | 20-07-2017 |
| | | | EP | 3192866 A1 | 19-07-2017 |
| | | | EP | 3402882 A1 | 21-11-2018 |
| | | | US | 2019062717 A1 | 28-02-2019 |
| | | | WO | 2017121902 A1 | 20-07-2017 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2017121902 A
- WO 2008005529 A
- WO 2009208941 A
- WO 2011109905 A

**Non-patent literature cited in the description**

- **ALONSO-LERMA, B. et al.** *Communications Materials*, 2020, vol. 1 (1), 57
- **KARLIN et al.** *Proc. Natl. Acad. Sci.*, 1990, vol. 87, 2264-8
- **KARLIN et al.** *Proc. Natl. Acad. Sci.*, 1993, vol. 90, 5873-7
- **ALTSCHUL et al.** *Nucleic Acids Res.*, 1991, vol. 25, 3389-402
- **ALTSCHUL et al.** *Nucleic Acids Res.*, 1997, vol. 25, 3389-402
- **ALTSCHUL et al.** *Methods in Enzymology*, 1996, vol. 266, 460-80
- *J. Mol. Biol.*, 1970, vol. 48, 444-53
- **MYERS** ; **MILLER**. *CABIOS*, 1989, vol. 4, 11-7
- **SMITH** ; **WATERMAN**. *Advances in Applied Mathematics*, 1981, vol. 2, 482-9
- **DORDO et al.** *J. Mol. Biol*, 1999, vol. 217, 721-739
- **TAYLOR et al.** *J. Theor. Biol.*, 1986, vol. 119, 205-218
- **KIM et al.** *Biotecnhology and bioprocess engineering*, 2013, vol. 19, 575-580
- **CAMPOS-OLIVER, A.** ; **QUIROZ-CASTAÑEDA, R** ; **ORTIZ-SURI, E. et al.** Cloning and expression of a hypothetical Loosenin from Neurospora crassa. *Rev Latinoam Biotecnol Ambient Algal*, 2014, vol. 5 (1), https://doi.org/10.7603/s40682-014-0001-y
- **SUZUKI H et al.** *Fungal Genet Biol*, November 2014, vol. 72, 115-123
- **DEEPIKA DAHIYA et al.** *Bioresource Technology*, 2024, vol. 394, 130188
- *CHEMICAL ABSTRACTS*, 9004-34-6
- **DA SILVA, T.A. et al.** *BioResources*, 2007, vol. 2 (4), 616-629
- **HU, J et al.** *Sci. Rep.*, 2018, vol. 8 (1), 3195
- **DAHIYA, D. et al.** *Bioresource Technology*, 2024, vol. 394, 130188